# EUROPEAN PATENT SPECIFICATION

(11) **EP 1 972 330 B1**
(45) Date of publication and mention of the grant of the patent: **12.09.2012**
(21) Application number: 08010779.0
(22) Date of filing: 13.06.2008
(51) Int. Cl.: A61K 8/898, A61Q 17/04, C08G 77/388, C08L 83/08

(54) **Cosmetic or pharmaceutical compositions comprising modified polysiloxanes with at least one carbamate group**
Kosmetische oder pharmazeutische Zusammensetzungen mit modifizierten Polysiloxanen mit mindestens einer Carbamatgruppe
Compositions cosmétiques ou pharmaceutiques comportant des polysiloxanes modifiés avec un groupe carbamate

(43) Date of publication of application: 24.09.2008
(73) Proprietor: Clariant Finance (BVI) Limited, Road Town, Tortola (VG)
(72) Inventor: Klug, Peter, Dr., 63762 Großostheim (DE); Pilz, Maurice, Frederic, Dr., 10405 Berlin (DE); Kluth, Guiseppina, 65779 Kelkheim (DE); Steffanut, Pascal, dr., 68128 Village-Neuf (FR); Meder, Markus, Dr., 79639 Grenzach-Wyhlen (DE)
(74) Representative: Paczkowski, Marcus

(56) References cited:
- EP-A- 0 639 369
- WO-A-99/03866
- US-A1- 2003 232 030

## Description

The invention relates to cosmetic or pharmaceutical compositions comprising one or more polysiloxanes having carbamate functions and their use as emulsifiers in the cosmetic industry.

There is literature on silicone compounds that are functionalized with nitrogen- (N-) containing groups. However, these compounds often differ significantly from silicone compounds comprising carbamate functions and are e.g. amino- or amido functional silicone compounds. These compounds are used for example as plasticizers in the textile industry, as surface treating agents, as thickeners or in the cosmetic industry. Nonetheless, there is a demand in the cosmetic and pharmaceutical industry for substances which e.g. can advantageously act as emulsifiers.

The object of the present invention was to provide cosmetic and pharmaceutical compositions comprising ingredients which are emulsifiable, compatible with additives and auxiliaries customary in cosmetic compositions, can be incorporated easily into formulations, produce the clearest possible appearance and can advantageously act as emulsifiers. Moreover, the compositions are to have good substantivity, exhibit a softening effect and bring about an improvement in the color absorption behavior and an increase in the color stability of tinted or colored hair.

Surprisingly, it has been found that this object is solved if one or more polysiloxanes of the general formula (I) 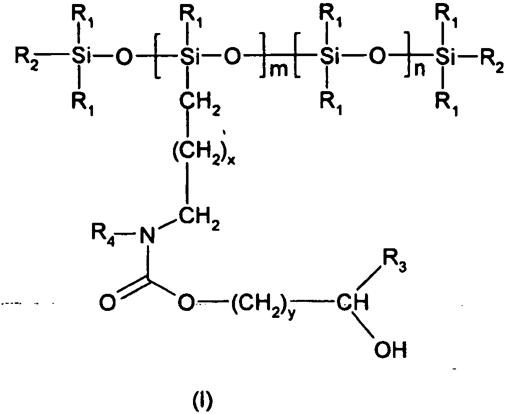 wherein
- R₁: are methyl residues;
- R₂: are methyl residues;
- R₃: is a -CH₂-OH residue;
- R₄: is -CH₂-(CH₂)_{w}-NH-C(=O)-O-(CH₂)_{z}-CH(OH)-R₅;
- R₅: is a -CH₂-OH residue;
- m: has a value of from 1 to 100 and preferably an average value of from 1 to 10;
- n: has a value of from 1 to 3000 and preferably an average value of from 40 to 600, particularly preferably an average value of from 40 to 350;
- w: is 1;
- x: is 1;
- y: is 1; and
- z: is 1
are incorporated in cosmetic or pharmaceutical compositions.

The present invention therefore relates to cosmetic or pharmaceutical compositions comprising one or more polysiloxanes of the general formula (I) 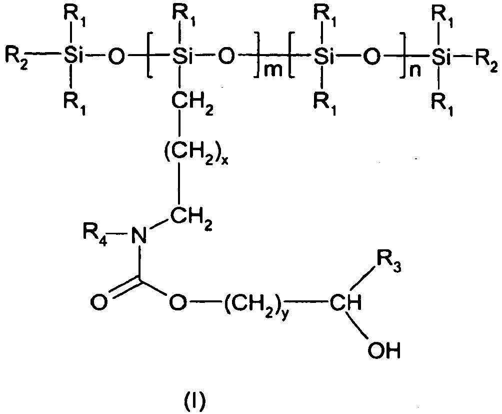 wherein
- R₁: are methyl residues;
- R₂: are methyl residues;
- R₃: is a -CH₂-OH residue;
- R₄: is -CH₂-(CH₂)_{w}-NH-C(=O)-O-(CH₂)_{z}-CH(OH)-R₅;
- R₅: is a -CH₂-OH residue;
- m: has a value of from 1 to 100 and preferably an average value of from 1 to 10;
- n: has a value of from 1 to 3000 and preferably an average value of from 40 to 600, particularly preferably an average value of from 40 to 350;
- w: is 1;
- x: is 1;
- y: is 1; and
- z: is 1.

The distribution of the Si-containing structure units of general formula (I) indexed with "m" and "n" over the polysiloxane chain can be statistical, random or blockwise.

The polysiloxanes of the general formula (I) are e.g. characterized in that they comprise at least one carbamate structure unit according to the following formula

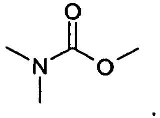

In the polysiloxanes of general formula (I) these carbamate structure units form part of one or more residues that are bonded to a polysiloxane chain. As can be seen from general formula (I) the carbamate comprising residues are not bonded terminally to the polysiloxane chain, i.e. they are not attached to one or both of the ends of the polysiloxane chain, but are attached laterally to the polysiloxane chain. Furthermore, and as also can be seen from general formula (I), the carbamate groups are attached to the polysiloxane chain via a bridging unit and their nitrogen atoms N.

EP 1 576 945 discloses cosmetic or pharmaceutical compositions comprising one or more substituted aminopolyorganosiloxanes. The amino groups present in these aminopolysiloxanes are substituted at least partially by a group that comprises a radical of a surfactant monoalcohol polyglycol ether with emulsifier character.

EP 1 754 468 discloses the use of polyquaternary polysiloxanes in cosmetic or pharmaceutical compositions for the cleaning and care of keratin fibers, in particular human hair. These polyquaternary polysiloxanes comprise nitrogen atoms N that bear four radicals and thus are positively charged.

US 4,104,296 describes organo-functional silicone components with functions according to the formula 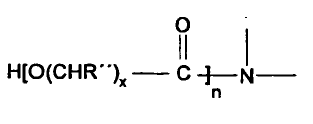 wherein R" is hydrogen and/or alkyl of 1 to 4 carbon atoms, x is about 2 to about 8 and n is at least 1. These components are e.g. useful to enhance the adhesion of various resins to inorganic substrates.

WO 2008/008077 describes functionalized polysiloxanes comprising carbamate groups. The carbamate groups of the polysiloxanes of WO 2008/008077 form part of residues that are bonded terminally, i.e. these residues are attached to the ends of the polysiloxane chain. The functionalized polysiloxanes of WO 2008/008077 may be useful as an antifouling material in marine environments.

WO 2007/024792 describes silicones comprising carbamate groups that are bonded to the ends of the silicone chain. The carbamate group comprising silicones of WO 2007/024792 may be used to produce adhesives.

KR 2007072069 describes polysiloxanes with laterally bonded residues that comprise a carbamate structure unit. These polysiloxanes e.g. may be used in fiber softening agents.

US 5,672,338 discloses hydroxyl carbamate functionalized silicones which may be used in personal care and cosmetic compositions. The carbamate groups of the hydroxyl carbamate functionalized silicones of US 5,672,338 are attached to the silicone chain via a bridging unit and their oxygen atoms O.

EP 0 639 369 discloses the conditioning of hair by applying to the hair an effective amount of a composition which includes as the hair conditioning agent, from 0.5 to 5.0 percent by weight of a derivatized amine functional organosilicon compound which is e.g. a reaction product of an amine functional polysiloxane and a derivative of carbonic acid such as ethylene carbonate or propylene carbonate.

The polysiloxanes of the general formula (I) are oils.

The color absorption behavior of hair colorants can be improved by the polysiloxanes of the general formula (I). In hairstyling compositions, a volumizing and shine-imparting effect of the polysiloxanes of the general formula (I) is also significant. Furthermore, the good compatibility with hydrophobic components, good dispersing and emulsifying power, the favorable viscosity behavior coupled with low viscosity and good incorporability in highly concentrated form, and a clear appearance of the polysiloxanes of the general formula (I) used according to the invention is advantageous. The polysiloxanes of the general formula (I) are characterized by good skin sensory properties and exhibit good spreadability, and an excellent gliding and carrier effect. Moreover, they are insensitive toward heat, UV radiation and IR radiation. They are thus valuable constituents of hair care and hair-cleansing compositions, hair colorants, skincare and skin-cleansing compositions, sunscreen compositions, deodorants, antiperspirants and decorative cosmetics.

The compositions according to the invention preferably comprise of from 0.01 to 10 % by weight, particularly preferably of from 0.1 to 5 % by weight and especially preferably of from 0.3 to 3 % by weight, based on the finished composition, of the one or more polysiloxanes of the general formula (I).

The compositions according to the invention may, for example, be aqueous, aqueous-alcoholic, aqueous-surface-active or alcoholic compositions, or compositions based on oil, inclusive compositions based on oil in anhydrous form, or emulsions, suspensions or dispersions.

In a preferred embodiment of the invention, the cosmetic or pharmaceutical compositions are in aqueous, aqueous-alcoholic, aqueous-surface-active or alcoholic form or represent compositions based on oil, inclusive anhydrous compositions based on oil, or are in the form of emulsions, suspensions or dispersions and, more particularly, in the form of fluids, foams, sprays, gels, mousses, lotions, creams or powders.

Using the polysiloxanes of the general formula (I) it is possible to prepare clear, viscous, aqueous, aqueous-alcoholic, aqueous-surface-active compositions, alcoholic compositions and also compositions based on oil with a very esthetic appearance.

In a further preferred embodiment of the invention, the cosmetic or pharmaceutical compositions are cosmetic or pharmaceutical formulations.

Further preferred embodiments of the compositions according to the invention are fluids, gels, oils, foams, sprays, lotions, cream gels, creams and powders.

Good substantivity, conditioning effect, and shine-imparting and volumizing effects of the polysiloxanes of the general formula (I) are utilized according to the invention for producing hair-treatment compositions, preferably shampoos, hair conditioners, hair treatments, styling compositions, hair rinses, volume sprays, styling fluids, hair foams, hair gels, setting compositions, hairsprays, mousses, hair oils and ends fluids.

In a further preferred embodiment of the invention, the cosmetic or pharmaceutical compositions thus are hair-treatment compositions, preferably shampoos, hair conditioners, hair treatments, styling compositions, hair rinses, volume sprays, styling fluids, hair foams, hair gels, setting compositions, hair sprays, mousses, hair oils and ends fluids.

The polysiloxanes of the general formula (I) improve the color absorption behavior of hair colorants and are thus valuable constituents in hair tints and colorants.

In a further preferred embodiment of the invention, the cosmetic or pharmaceutical compositions thus are hair tints and/or colorants.

Conditioning effects and good skin sensory properties of skincare compositions and skin-cleansing compositions are achieved by the polysiloxanes of the general formula (I).

In a further preferred embodiment of the invention, the cosmetic or pharmaceutical compositions are rinse-off products, in particular shower baths, shower gels or foam baths.

In a further preferred embodiment of the invention, the cosmetic or pharmaceutical compositions are leave-on products, in particular day creams, night creams, care creams, nutrient creams, body lotions, ointments or lipcare compositions. Among these compositions day and night creams are particularly preferred. Preferably the inventive day and night creams comprise one or more substances selected from organic and inorganic UV absorbers.

Further preferred leave-on products are decorative cosmetics, in particular make-ups, eyeshadows, lipsticks or mascara. These products comprise one or more substances selected from the group consisting of pigments, colorants, tints, dyes, pearlescent imparting substances and glitter imparting substances.

In a further preferred embodiment of the invention, the cosmetic or pharmaceutical compositions are sunscreen compositions. These compositions comprise one or more substances selected from the group consisting of inorganic and organic UV absorbers.

The inventive sunscreen compositions preferably are sunscreen formulations, particularly preferably sunscreen milks, sunscreen creams or sunscreen blockers.

In a further preferred embodiment of the invention, the cosmetic or pharmaceutical compositions are deodorants and antiperspirants, in particular in the form of sprays, sticks, gels or lotions.

In a further preferred embodiment of the invention, the cosmetic or pharmaceutical compositions are surfactant-free compositions, in particular surfactant-free solid compositions or surfactant-free emulsions.

In a further preferred embodiment of the invention, the cosmetic or pharmaceutical compositions are permanent waving compositions, in particular conditioners.

In a particularly preferred embodiment of the invention, the cosmetic or pharmaceutical compositions are aqueous or aqueous-alcoholic cosmetic or pharmaceutical compositions.

In a further particularly preferred embodiment of the invention, the cosmetic or pharmaceutical compositions are anhydrous compositions based on oils.

In a further particularly preferred embodiment of the invention, the cosmetic or pharmaceutical compositions are emulsions.

The inventive emulsions may either be water-in-oil emulsions or oil-in-water emulsions, microemulsions, nanoemulsions and multiple emulsions. The inventive emulsions can be prepared in a known manner, i.e. for example by cold, hot, hot/cold or PIT emulsification.

The inventive emulsions preferably are water-in-oil- (W/O-) emulsions or oil-in-water-(O/W-) emulsions, particularly preferably water-in-oil-emulsions.

The inventive oil-in-water emulsions preferably comprise water or the aqueous fraction in an amount of from 5 to 95 % by weight, preferably 15 to 75 % by weight, particularly preferably 25 to 85 % by weight, based on the finished compositions.

The inventive water-in-oil emulsions preferably comprise the oil fraction in an amount of from 5 to 95 % by weight, preferably 15 to 75 % by weight, particularly preferably 25 to 65 % by weight, based on the finished compositions.

In a preferred embodiment of the invention the inventive W/O-emulsions are water-in-silicone emulsions (W/Si emulsions). These inventive water-in-silicone emulsions preferably comprise the silicone fraction in an amount of from 5 to 95 % by weight, preferably 15 to 75 % by weight, particularly preferably 25 to 65 % by weight, based on the finished compositions.

In a further preferred embodiment of the invention the inventive O/W-emulsions are silicone-in-water emulsions (Si/W emulsions). These inventive silicone-in-water emulsions preferably comprise water or the aqueous fraction in an amount of from 5 to 95 % by weight, preferably 15 to 75 % by weight, particularly preferably 25 to 85 % by weight, based on the finished compositions.

For the compositions according to the invention on an aqueous-alcoholic or alcoholic basis, all mono- or polyhydric alcohols are suitable. Preference is given to alcohols having 1 to 4 carbon atoms, such as ethanol, propanol, isopropanol, n-butanol, isobutanol, t-butanol or glycerol, and alkylene glycols, in particular propylene glycol, butylene glycol or hexylene glycol, and mixtures of said alcohols. Further preferred alcohols are polyethylene glycols with a relative molecular mass below 2000. In particular, a use of polyethylene glycol with a relative molecular mass between 200 and 600 and of polyethylene glycol with a relative molecular mass between 400 and 600 is preferred.

The oil-based compositions according to the invention can preferably comprise: hydrocarbon oils with linear or branched, saturated or unsaturated C₇-C₄₀-carbon chains, for example dodecane, isododecane, cholesterol, hydrogenated polyisobutylenes, docosanes, hexadecane, isohexadecane, paraffins and isoparaffins, but also triglycerides of animal and vegetable origin, for example beef tallow, pig fat, goose grease, perhydrosqualene, lanolin, sunflower oil, maize oil, soy bean oil, rice oil, jojoba oil, babusscu oil, pumpkin oil, grape seed oil, sesame oil, walnut oil, apricot oil, macadamia oil, avocado oil, sweet almond oil, lady's smock oil, castor oil, olive oil, peanut oil, rape seed oil and coconut oil and synthetic oils, such as purcellin oil, linear and/or branched fatty alcohols and fatty acid esters, preferably Guerbet alcohols having 6 to 18, preferably 8 to 10, carbon atoms; esters of linear (C₆-C₁₃)-fatty acids with linear (C₆-C₂₀)-fatty alcohols; esters of branched (C₆-C₁₃)-carboxylic acids with linear (C₆-C₂₀)-fatty alcohols, esters of linear (C₆-C₁₈)-fatty acids with branched alcohols, in particular 2-ethylhexanol; esters of linear and/or branched fatty acids with polyhydric alcohols (such as e.g. dimerdiol or trimerdiol) and/or Guerbet alcohols; alcohol esters of C₁-C₁₀-carboxylic acids or C₂-C₃₀-dicarboxylic acids, esters, such as dioctyl adipate, diisopropyl dimer dilineolate; propylene glycols/dicaprylate or waxes, such as beeswax, paraffin wax or microcrystalline waxes, optionally in combination with hydrophilic waxes, such as, for example, cetylstearyl alcohol; fluorinated and perfluorinated oils; monoglycerides of C₁-C₃₀-carboxylic acids, diglycerides of C₁-C₃₀-carboxylic acids, triglycerides of C₁-C₃₀-carboxylic acids, for example triglycerides of caprylic/capric acids, ethylene glycol monoesters of C₁-C₃₀-carboxylic acids, ethylene glycol diesters of C₁-C₃₀-carboxylic acids, propylene glycol monoesters of C₁-C₃₀-carboxylic acids, propylene glycol diesters of C₁-C₃₀-carboxylic acids, and propoxylated and ethoxylated derivatives of the abovementioned classes of compound. The carboxylic acids can comprise linear or branched alkyl groups or aromatic groups. By way of example, mention may be made of diisopropyl sebacate, diisopropyl adipate, isopropyl myristate, isopropyl palmitate, myristyl propionate, ethylene glycol distearate, 2-ethylhexyl palmitate, isodecyl neopentanoate, di-2-ethylhexyl maleate, cetyl palmitate, myristyl myristate, stearyl stearate, cetyl stearate, behenyl behenate, dioctyl maleate, dioctyl sebacate, cetyl octanoate, diisopropyl dilinoleate, caprylic/capryl triglyceride, PEG-6 caprylic/capryl triglyceride, PEG-8 caprylic/capryl triglyceride, cetyl ricinoleate, cholesterol hydroxystearate, cholesterol isostearate, C₁-C₃₀-monoesters and polyesters of glycerol, for example glyceryl tribehenate, glyceryl stearate, glyceryl palmitate, glyceryl distearate, glyceryl dipalmitate, C₁-C₃₀-carboxylic monoesters and polyesters of sugars, for example glucose tetraoleate, glucose tetraesters of soy bean oil fatty acid, mannose tetraesters of soy bean oil fatty acid, galactose tetraesters of oleic acid, arabinose tetraesters of linoleic acid, xylose tetralinoleate, galactose pentaoleate, sorbitol tetraoleate, sorbitol hexaesters of unsaturated soy bean oil fatty acid, xylitol pentaoleate, sucrose tetraoleate, sucrose pentaoleate, sucrose hexaoleate, sucrose heptaoleate, sucrose oleate.

The silicone oils available are preferably dimethylpolysiloxanes and cyclomethicones, polydialkylsiloxanes R₃SiO(R₂SiO)ₓSiR₃, where R is methyl or ethyl, particularly preferably methyl, and x is a number from 2 to 500, for example the dimethicones available under the trade names VICASIL (General Electric Company), DOW CORNING 200, DOW CORNING 225, DOW CORNING 200 (Dow Corning Corporation), trimethylsiloxysilicates [(CH₂)₃SiO)_{1/2}]ₓ[SiO₂]_{y}, where x is a number from 1 to 500 and y is a number from 1 to 500, dimethiconols R₃SiO[R₂SiO]ₓSiR₂OH and HOR₂SiO[R₂SiO]ₓSiR₂OH, where R is methyl or ethyl and x is a number up to 500, polyalkylarylsiloxanes, for example the polymethylphenylsiloxanes available under the trade names SF 1075 METHYLPHENYL FLUID (General Electric Company) and 556 COSMETIC GRADE PHENYL TRIMETHICONE FLUID (Dow Corning Corporation), polydiarylsiloxanes, silicone resins, cyclic silicones and amino-, fatty acid-, alcohol-, polyether-, epoxy-, fluorine- and/or alkyl-modified silicone compounds, polyether siloxane copolymers, Trimethicones, for example Phenyltrimethicone SilCare^{®} Silicone 15M50 and Caprylyl Trimethicone 31M50 and Methicones, for examples Caprylyl Methicone SilCare^{®} Silicone 41M15.

The colorants and tints, preferably the hair colorants and tints according to the invention preferably comprise direct dyes and/or oxidation dye precursors in the customary pH ranges. Suitable direct dyes are preferably nitroaniline derivatives, such as 1-[(2-hydroxyethyl)amino]-2-nitrobenzene (Velsol^{®} Yellow 2), 4-hydroxypropylamino-3-nitrophenol (Velsol^{®} Red BN), 3-nitro-p-hydroxyethylaminophenol (Velsol^{®} Red 54), 4-hydroxyethylamino-3-nitroaniline (Velsol^{®} Red 3), N,N'-bis(hydroxyethyl)-2-nitro-p-phenylenediamine (Velsol^{®} Violet BS), N,N',N'-tris(hydroxyethyl)-2-nitro-p-phenylenediamine (Velsol^{®} Blue 2), 4-(2'-hydroxyethyl)amino-3-nitrotoluene, 4-(2'-hydroxyethyl)amino-3-nitrobenzyl alcohol, 4-(2'-hydroxyethyl)amino-3-nitro-1-trifluoromethylbenzene, 4-(2',3'-dihydroxypropyl)amino-3-nitrochlorobenzene, 4-(2'-hydroxyethyl)amino-3-nitrobromobenzene and 4-(2',3'-dihydroxypropyl)amino-3-nitrobromobenzene, nitrobenzene derivatives, for example 2-amino-4-nitrophenol, picramic acid, 1-[(2'-hydroxyethyl)amino]-2-amino-4-nitrobenzene, 2-nitro-4-[(2'-hydroxyethyl)amino]aniline, 4-bis[(2'-hydroxyethyl)amino]-1-methylamino-2-nitrobenzene, 2,5-bis[(2'-hydroxyethyl)amino]nitrobenzene, 2-(2'-hydroxyethyl)amino-4,6-dinitrophenol, 1-amino-4-(2',3'-dihydroxypropyl)amino-2-nitro-5-chlorobenzene, but also triphenylmethane dyes such as, for example, Basic Violet 1 (C.I. 42535), azodyes, such as, for example, Acid Brown 4 (C.I. 14805), anthraquinone dyes such as, for example, Disperse Blue 23 (C.I. 61545), Disperse Violet 4 (C.I. 61105), 1,4,5,8-tetraaminoanthraquinone and 1,4-diaminoanthraquinone and further direct dyes.

Oxidation dye precursors which are available are preferably p-phenylenediamines and p-aminophenols and derivatives thereof, such as, for example, p-tolylenediamine, p-phenylenediamine, p-aminophenol, which are combined with so-called modifiers or couplers, such as, for example, m-phenylenediamine, resorcinol, m-aminophenol and derivatives thereof for the purpose of shading the coloration.

Suitable oxidizing agents for developing the hair colorations are preferably hydrogen peroxide and its addition compounds.

To increase the color intensity, the compositions according to the invention can comprise the carriers customary in cosmetic systems, in particular benzyl alcohol, vanillin (4-hydroxy-3-methoxybenzaldehyde), isovanillin, p-hydroxyanisol, 3-hydroxy-4-methoxybenzaldehyde, 2-phenoxyethanol, salicylaldehyde, 3,5-dihydroxybenzaldehyde, 3,4-dihydroxybenzaldehyde, 4-hydroxyphenylacetamide, methyl p-hydroxybenzoate, p-hydroxybenzaldehyde, m-cresol, hydroquinone monomethyl ether, o-fluorophenol, m-fluorophenol, p-fluorophenol, 2-(2'-hydroxyphenoxy)ethanol, 3,4-methylenedioxyphenol, resorcinol monomethyl ether, 3,4-dimethoxyphenol, 3-trifluoromethylphenol, resorcinol monoacetate, ethylvanillin, 2-thiophenethanol, butyl lactate and butyl glycolate. Of particular advantage with a synergistic effect are compositions according to the invention comprising phenoxyethanol and/or benzyl alcohol.

The hair colorants according to the invention can advantageously comprise pearlescence-imparting compounds, for example fatty acid monoalkanolamides, fatty acid dialkanolamides, monoesters or diesters of alkylene glycol, in particular ethylene glycol and/or propylene glycol or oligomers thereof with higher fatty acids, e.g. palmitic acid, stearic acid or behenic acid, or mixtures thereof, monoesters or diesters of alkylene glycols with fatty acids, fatty acids and metal salts thereof, monoesters or polyesters of glycerol with carboxylic acids and ketosulfones of various types, preferably ethylene glycol distearate and polyethylene glycol distearate with about 3-glycol units.

The hair-treatment compositions according to the invention preferably comprise 0.1 to 15 % by weight, particularly preferably 1 to 10 % by weight, of pearlescence-imparting compounds, based on the finished compositions.

Glitter and shine effects of the compositions according to the invention can be produced preferably by adding mica, colored polyacrylic esters and mica, mica-iron oxide, mica-titanium oxide and through pigments. Suitable pigments are metal oxides, for example iron oxides, titanium oxide, ultramarine blue, and pigments modified with cationic coating shells, as described in WO 00/12053 and EP 504 066.

As further auxiliaries and additives, the cosmetic compositions according to the invention can comprise surfactants, emulsifiers, cationic polymers, thickeners, film formers, antimicrobial active ingredients, astringents, antioxidants, UV light protection filters, pigments/micropigments, gelling agents, and further additives customary in cosmetics, such as, for example, superfatting agents, moisturizing agents, silicones, stabilizers, conditioning agents, glyceryol, preservatives, pearlizing agents, dyes, fragrance and perfume oils, solvents, hydrotropes, opacifiers, fatty alcohols, substances with a keratolytic and keratoplastic effect, antidandruff agents, biogenic active ingredients (local anesthetics, antibiotics, antiphlogistics, antiallergics, corticosteroids, sebostatics), vitamins, Bisabolol^{®}, Allantoin^{®}, Phytantriol^{®}, Panthenol^{®}, AHA acids (α-hydroxy carboxylic acids), plant extracts, for example aloe vera and proteins.

Anionic washing-active substances which may be mentioned are preferably: C₁₀-C₂₀-alkyl and alkylene carboxylates, alkyl ether carboxylates, fatty alcohol sulfates, fatty alcohol ether sulfates, alkylamide sulfates and sulfonates, fatty acid alkylamide polyglycol ether sulfates, alkanesulfates, alkanesulfonates, and hydroxyalkanesulfonates, olefinsulfonates, acylesters of isothionates, α-sulfo fatty acid esters, alkylbenzenesulfonates, alkylphenol glycol ether sulfonates, sulfosuccinates, sulfosuccinic monoesters and diesters, fatty alcohol ether phosphates, protein-fatty acid condensation products, alkyl monoglyceride sulfates and sulfonates, alkyl glyceride ether sulfonates, fatty acid methyl taurides, fatty acid sarcosinates, sulforicinoleates, amphoacetates or amphoglycinates, acylglutamates. These compounds and their mixtures are used in the form of their water-soluble or water-dispersible salts, for example the sodium, potassium, magnesium, ammonium, mono-, di- and triethanolammonium and analogous alkylammonium salts.

The weight fraction of the anionic surfactants is preferably 1 to 30 % by weight, particularly preferably 5 to 25 % by weight, especially preferably 10 to 22 % by weight, based on the finished compositions.

Suitable cationic surfactants are, for example, quaternary ammonium salts, such as di(C₁₀-C₂₄-alkyl)dimethylammonium chloride or bromide, preferably di(C₁₂-C₁₈-alkyl)dimethylammonium chloride or bromide; C₁₀-C₂₄-alkyldimethylethylammonium chloride or bromide C₁₀-C₂₄ alkyltrimethylammonium chloride or bromide, preferably cetyltrimethylammonium chloride or bromide and C₂₀-C₂₂-alkyltrimethylammonium chloride or bromide; C₁₀-C₂₄-alkyldimethylbenzylammonium chloride or bromide, preferably C₁₂-C₁₈-alkyldimethylbenzylammonium chloride; N-(C₁₀-C₁₈-alkyl)pyridinium chloride or bromide, preferably N-(C₁₂-C₁₆-alkyl)pyridinium chloride or bromide; N-(C₁₀-C₁₈-alkyl)isoquinolinium chloride, bromide or monoalkylsulfate; N-(C₁₂-C₁₈-alkyl)polyoylaminoformylmethyl)pyridinium chloride; N- (C₁₂-C₁₈-alkyl)-N-methylmorpholinium chloride, bromide or monoalkylsulfate; N-(C₁₂-C₁₈-alkyl)-N-ethylmorpholinium chloride, bromide or monoalkylsulfate; C₁₆-C₁₈-alkylpentaoxyethylammonium chloride; diisobutylphenoxyethoxyethyldimethylbenzylammonium chloride; salts of N, N-diethylaminoethylstearylamide and -oleylamide with hydrochloric acid, acetic acid, lactic acid, citric acid, phosphoric acid; N-acylaminoethyl, N,N-diethyl-N-methylammonium chloride, bromide or monoalkylsulfate and N-acylaminoethyl-N,N-diethyl-N-benzylammonium chloride, bromide or monoalkylsulfate, where acyl is preferably stearyl or oleyl.

The weight fraction of the cationic surfactants is preferably 0.1 to 10 % by weight, particularly preferably 0.2 to 7 % by weight, especially particularly preferably 0.5 to 5 % by weight, based on the finished composition.

Suitable nonionic surfactants which can be used as washing-active substances are preferably fatty alcohol ethoxylates (alkylpolyethylene glycols); alkylphenol polyethylene glycols; alkyl mercaptan polyethylene glycols; fatty amine ethoxylates (alkylaminopolyethylene glycols); fatty acid ethoxylates (acyl polyethylene glycols); polypropylene glycol ethoxylates (Pluronics^{®}); fatty acid amide polyethylene glycols; N-alkyl-, N-alkoxypolyhydroxy fatty acid amide, in particular fatty acid N-methylglucamides, sucrose esters; polyglycol ethers, alkyl polyglycosides, phosphoric esters (mono-, di- and triphosphoric esters ethoxylated and nonethoxylated).

The weight fraction of the nonionic surfactants in the compositions according to the invention (e.g. in the case of rinse-off products) is preferably in the range from 1 to 20 % by weight, particularly preferably 2 to 10% by weight, especially preferably 3 to 7 % by weight, based on the finished composition.

Preferred amphoteric surfactants are: N-(C₁₂-C₁₈-alkyl)-β-aminopropionates and N-(C₁₂-C₁₈-alkyl)-β-iminodipropionates as alkali metal and mono-, di- and trialkylammonium salts; N-acylaminoalkyl-N,N-dimethylacetobetaine, preferably N-(C₈-C₁₈-acyl)aminopropyl-N,N-dimethylacetobetaine; C₁₂-C₁₈-alkyldimethylsulfopropylbetaine; amphoteric surfactants based on imidazoline (trade name: Miranol^{®}, Steinapon^{®}), preferably the sodium salt of 1-β-carboxymethyloxyethyl)-1-(carboxymethyl)-2-laurylimidazolinium; amine oxides, e.g. C₁₂-C₁₈-alkyldimethylamine oxide, fatty acid amidoalkyldimethylamine oxide.

The weight fraction of the amphoteric surfactants is preferably 0.5 to 20 % by weight, particularly preferably 1 to 10 % by weight, based on the finished composition.

Furthermore, foam-boosting co-surfactants from the group consisting of alkylbetaines, alkylamidobetaines, aminopropionates, aminoglycinates, imidazoliniumbetaines and sulfobetaines, amine oxides and fatty acid alkanolamides or polyhydroxyamides can be used in the compositions according to the invention.

Preferred surfactants in the compositions according to the invention are alkyl ether sulfates, alkylsulfates, in particular laurylsulfate, alkylbetaines, in particular cocoamidopropylbetaine, amphoacetates, acylglutamates, in particular sodium cocoylglutamate, alkyl ether sulfosuccinates, in particular disodium laureth sulfosuccinate and coconut fatty acid diethanolamide.

The total amount of the surfactants used in the compositions according to the invention is preferably 1 to 70 % by weight, particularly preferably 10 to 40 % by weight, especially preferably 12 to 35 % by weight, based on the finished composition.

Compositions according to the invention in the form of emulsions can be produced without further emulsifier or else comprise one or more emulsifiers. These emulsifiers can be chosen from the group of nonionic, anionic, cationic or amphoteric emulsifiers.

Suitable nonionogenic co-emulsifiers are preferably addition products of from 0 to 30-mol of ethylene oxide and/or 0 to 5 mol of propylene oxide onto linear fatty alcohols having 8 to 22 carbon atoms, onto fatty acids having 12 to 22 carbon atoms, only alkylphenols having 8 to 15 carbon atoms in the alkyl group and onto sorbitan or sorbitol esters; (C₁₂-C₁₈) fatty acid monoesters and diesters of addition products of from 0 to 30-mol of ethylene oxide onto glycerol; glycerol monoesters and diesters and sorbitan monoesters and diesters of saturated and unsaturated fatty acids having 6 to 22 carbon atoms and optionally ethylene oxide addition products thereof; addition products of from 15 to 60-mol of ethylene oxide onto castor oil and/or hydrogenated castor oil; polyol and, in particular, polyglycerol, esters, such as, for example, polyglycerol polyricinoleate and polyglycerol poly-12-hydroxystearate. Likewise preferably suitable are ethoxylated fatty amines, fatty acid amides, fatty acid alkanolamides and mixtures of compounds of two or more of these classes of substance.

Suitable ionogenic coemulsifiers are, for example, anionic emulsifiers, such as mono-, di- or triphosphoric esters, soaps (e.g. sodium stearate), fatty alcohol sulfates, but also cationic emulsifiers, such as mono-, di- and trialkylquats and polymeric derivatives thereof.

Available amphoteric emulsifiers are preferably alkylaminoalkylcarboxylic acids, betaines, sulfobetaines and imidazoline derivatives.

It is also possible to use naturally occurring emulsifiers, of these preference being given to beeswax, wool wax, lecithin and sterols.

Fatty alcohol ethoxylates are preferably chosen from the group of ethoxylated stearyl alcohols, cetyl alcohols, cetylstearyl alcohols, in particular polyethylene glycol(13) stearyl ether, polyethylene glycol(14) stearyl ether, polyethylene glycol(15) stearyl ether, polyethylene glycol(16) stearyl ether, polyethylene glycol(17) stearyl ether, polyethylene glycol(18) stearyl ether, polyethylene glycol(19) stearyl ether, polyethylene glycol(20) stearyl ether, polyethylene glycol(12) isostearyl ether, polyethylene glycol(13) isostearyl ether, polyethylene glycol(14) isostearyl ether, polyethylene glycol(15) isostearyl ether, polyethylene glycol(16) isostearyl ether, polyethylene glycol(17) isostearyl ether, polyethylene glycol(18) isostearyl ether, polyethylene glycol(19) isostearyl ether, polyethylene glycol(20) isostearyl ether, polyethylene glycol(13) cetyl ether, polyethylene glycol(14) cetyl ether, polyethylene glycol(15) cetyl ether, polyethylene glycol(16) cetyl ether, polyethylene glycol(17) cetyl ether, polyethylene glycol(18) cetyl ether, polyethylene glycol(19) cetyl ether, polyethylene glycol(20) cetyl ether, polyethylene glycol(13) isocetyl ether, polyethylene glycol(14) isocetyl ether, polyethylene glycol(15) isocetyl ether, polyethylene glycol(16) isocetyl ether, polyethylene glycol(17) isocetyl ether, polyethylene glycol(18) isocetyl ether, polyethylene glycol(19) isocetyl ether, polyethylene glycol(20) isocetyl ether, polyethylene glycol(12) oleyl ether, polyethylene glycol(13) oleyl ether, polyethylene glycol(14) oleyl ether, polyethylene glycol(15) oleyl ether, polyethylene glycol(12) lauryl ether, polyethylene glycol(12) isolauryl ether, polyethylene glycol(13) cetylstearyl ether, polyethylene glycol(14) cetylstearyl ether, polyethylene glycol(15) cetylstearyl ether, polyethylene glycol(16) cetylstearyl ether, polyethylene glycol(17) cetylstearyl ether, polyethylene glycol(18) cetylstearyl ether, polyethylene glycol(19) cetylstearyl ether, polyethylene glycol(20) cetylstearyl ether, polyethylene glycol(20) stearate, polyethylene glycol(21) stearate, polyethylene glycol(22) stearate, polyethylene glycol(23) stearate, polyethylene glycol(24) stearate, polyethylene glycol(25) stearate, polyethylene glycol(12) isostearate, polyethylene glycol(13) isostearate, polyethylene glycol(14) isostearate, polyethylene glycol(15) isostearate, polyethylene glycol(16) isostearate, polyethylene glycol(17) isostearate, polyethylene glycol(18) isostearate, polyethylene glycol(19) isostearate, polyethylene glycol(20) isostearate, polyethylene glycol(21) isostearate, polyethylene glycol(22) isostearate, polyethylene glycol(23) isostearate, polyethylene glycol(24) isostearate, polyethylene glycol(25) isostearate, polyethylene glycol(12) oleate, polyethylene glycol(13) oleate, polyethylene glycol(14) oleate, polyethylene glycol(15) oleate, polyethylene glycol(16) oleate, polyethylene glycol(17) oleate, polyethylene glycol(18) oleate, polyethylene glycol(19) oleate, polyethylene glycol(20) oleate.

As ethoxylated alkyl ether carboxylic acid or salts thereof it is advantageously possible to use sodium laureth 11-carboxylate.

An advantageous alkyl ether sulfate is sodium laureth-14 sulfate, and an advantageous ethoxylated cholesterol derivative is polyethylene glycol(30) cholesteryl ether. Preference is likewise given to polyethylene glycol(25) soyasterol.

Ethoxylated triglycerides which can be used advantageously are polyethylene glycol(60) evening primrose glycerides.

It is also advantageous to choose the polyethylene glycol glycerol fatty acid esters from the group consisting of polyethylene glycol(20) glyceryl laurate, polyethylene glycol(6) glyceryl caprate, polyethylene glycol(20) glyceryl oleate, polyethylene glycol(20) glyceryl isostearate and polyethylene glycol(18) glyceryl oleate/cocoate.

Among the sorbitan esters, polyethylene glycol(20) sorbitan monolaurate, polyethylene glycol(20) sorbitan monostearate, polyethylene glycol(20) sorbitan monoisostearate, polyethylene glycol(20) sorbitan monopalmitate, polyethylene glycol(20) sorbitan monooleate are particularly suitable.

Advantageous W/O emulsifiers which can be used are the following: fatty alcohols having 8 to 30 carbon atoms, monoglycerol esters of saturated and/or unsaturated, branched and/or unbranched alkanecarboxylic acids with a chain length of from 8 to 24, in particular 12 to 18, carbon atoms, diglycerol esters of saturated and/or unsaturated, branched and/or unbranched alkanecarboxylic acids with a chain length of from 8 to 24, in particular 12 to 18, carbon atoms, monoglycerol ethers of saturated and/or unsaturated, branched and/or unbranched alcohols with a chain length of from 8 to 24, in particular 12 to 18, carbon atoms, diglycerol ethers of saturated and/or unsaturated, branched and/or unbranched alcohols with a chain length of from 8 to 24, in particular 12 to 18, carbon atoms, propylene glycol esters of saturated and/or unsaturated, branched and/or unbranched alkanecarboxylic acids of chain length from 8 to 24, in particular 12 to 18, carbon atoms, and sorbitan esters of saturated and/or unsaturated, branched and/or unbranched alkanecarboxylic acids with a chain length of from 8 to 24, in particular 12 to 18, carbon atoms.

Particularly advantageous W/O emulsifiers are glyceryl monostearate, glyceryl monoisostearate, glyceryl monomyristate, glyceryl monooleate, glyceryl monolaurate, glyceryl monocaprylate, glyceryl monocaprate, diglyceryl monostearate, diglyceryl monoisostearate, propylene glycol monostearate, propylene glycol monoisostearate, propylene glycol monocaprylate, propylene glycol monolaurate, sorbitan monoisostearate, sorbitan monolaurate, sorbitan monocaprylate, sorbitan monoisooleate, sucrose distearate, cetyl alcohol, stearyl alcohol, arachidyl alcohol, behenyl alcohol, isobehenyl alcohol, selachyl alcohol, chimyl alcohol or polyethylene glycol(2) stearyl ether.

The weight fraction of the emulsifier or emulsifiers present in the compositions according to the invention, in addition to the polysiloxanes of the general formula (I) is preferably 0.1 to 20 % by weight, particularly preferably 0.5 to 15 % by weight, especially preferably 1 to 10 % by weight, based on the finished composition.

Suitable cationic polymers are preferably the compounds known under the INCl name "Polyquaternium", in particular Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, Polyquaternium-37&mineral oil&PPG trideceth (Salcare^{®} SC95), PVP dimethylaminoethyl methacrylate copolymer, guar hydroxypropyltriammonium chlorides, and calcium alginate and ammonium alginate.

Furthermore, the following may preferably be used: cationic cellulose derivatives; cationic starch; copolymers of diallylammonium salts and acrylamides; quaternized vinylpyrrolidone/vinylimidazole polymers; condensation products of polyglycols and amines; quaternized collagen polypeptides; quaternized wheat polypeptides; polyethyleneimines; cationic silicone polymers, such as, for example, amidomethicones; copolymers of adipic acid and dimethylaminohydroxypropyldiethylenetriamine; polyaminopolyamide and cationic chitin derivatives, such as, for example, chitosan.

The weight fraction of cationic polymers in the compositions according to the invention can preferably be in the range from 0.1 to 10 % by weight, particularly preferably in the range from 0.2 to 5 % by weight, especially preferably in the range from 0.5 to 2.5 % by weight, based on the finished compositions.

The desired viscosity of the compositions can be adjusted by adding thickeners. Of suitability are preferably cellulose ethers and other cellulose derivatives (e.g. carboxymethylcellulose, hydroxyethylcellulose), gelatin, starch and starch derivatives, sodium alginates, fatty acid polyethylene glycol esters, agar agar, traganth or dextrin derivatives, in particular dextrin esters.

The synthetic polymers used are various materials, preferably polyvinyl alcohols, polyacrylamides, polyvinylamides, polysulfonic acids, in particular copolymers based on ammonium salts of acrylamidoalkylsulfonic acids and cyclic N-vinylcarboxamides or cyclic and linear N-vinylcarboxamides and also hydrophobically modified acrylamidoalkylsulfonic acid copolymers, polyacrylic acid, polyacrylic acid derivatives, polyacrylic esters, polyvinylpyrrolidone, polyvinyl methyl ether, polyethylene oxides, copolymers of maleic anhydride and vinyl methyl ether, and various mixtures and copolymers of the abovementioned compounds, including their various salts and esters. These polymers can, if desired, be crosslinked or uncrosslinked.

Thickeners which are particularly suitable especially for oil-based compositions are dextrin esters, for example dextrin palmitate, but also fatty acid soaps, fatty alcohols and silicone waxes, for example alkylmethicones, SilCare^{®} Silicone 41M40, SilCare^{®} Silicone 41M50 and alkyldimethicones, SilCare^{®} Silicone 41M65, SilCare^{®} Silicone 41M70, SilCare^{®} Silicone 41M80 or SilCare^{®} Silicone 41M90.

Depending on the intended use, preferred film formers are salts of phenylbenzimidazolesulfonic acid, water-soluble polyurethanes, for example C₁₀-polycarbamylpolyglyceryl esters, polyvinyl alcohol, polyvinylpyrrolidone copolymers, for example vinylpyrrolidone/vinyl acetate copolymer, water-soluble acrylic acid polymers/copolymers or esters or salts thereof, for example partial ester copolymers of acrylic/methacrylic acid and polyethylene glycol ethers of fatty alcohols, such as acrylate/steareth-20 methacrylate copolymer, water-soluble cellulose, for example hydroxymethylcellulose, hydroxyethylcellulose, hydroxypropylcellulose, water-soluble quaterniums, polyquaterniums, carboxyvinyl polymers, such as carbomers and salts thereof, polysaccharides, for example polydextrose and glucan, vinyl acetate/crotonate, available for example under the trade name Aristoflex^{®} A 60 (Clariant), and polymeric amine oxides, for example representatives available under the trade names Diaformer Z-711, 712, 731, 751.

In a preferred embodiment of the invention, the cosmetic or pharmaceutical compositions comprise one or more antimicrobial active ingredients.

Preferably suitable antimicrobial active ingredients such as preservatives or fungicidal active ingredients are alcohols containing one or more aromatic substituents, preferably 2-phenoxyethanol, 1-phenoxy-2-propanol, benzylalcohol, 2-hydroxybiphenyl and 2-phenoxyethanol, parabens, preferably methylparaben, ethylparaben, propylparaben, butylparaben, isobutylparaben, sodium methylparaben, sodium ethylparaben, sodium propylparaben, sodium isobutylparaben, sodium butylparaben or sodium isobutylparaben, formaldehyde donating actives, preferably imidazolidinyl urea, diazolidinyl urea, sodium hydroxymethylglycinate, DMDM hydantoin, halogenated actives, preferably iodopropynyl butylcarbamate, 2-bromo-2-nitropropan-1,3-diol, 2,4,4'-trichloro-2'-hydroxydiphenylether (triclosan), 3,4,4'-trichlorocarbanilide (triclocarban), chlorbutanulum, 2,4-dichlorobenzyl alcohol, N-(4-chlorphenyl-N'-(3,4-dichlorphenyl)urea, 1,2-dibromo-2,4-dicyanobutane, methylchloroisothiazolinone and methylisothiazolinone in the molar ratio of 3:1, chloroxylenol, ketoconazole, oxiconazole, butoconazole, clotrimazole, econazole, enilconazole, fenticonazole, miconazole, sulconazole, tioconazole, fluconazole, itraconazole, terconazole, cationic nitrogen containing actives, preferably cetyltrimethylammonium chloride, cetylpyridinium chloride, benzethonium chloride, diisobutylethoxyethyl-dimethylbenzylammonium chloride, diisobutyl-phenoxy-ethoxyethyl-dimethylbenzyl-ammonium chloride, N-alkyl-N,N-dimethyl-benzyl-ammonium chloride, -bromide, -saccharinate, trimethylammonium chloride, sodium aluminiumchlorohydroxylacetate, tricetylmethylammonium chloride, diaminoalkylamide, for example L-lysinhexadecylamide, organic acids and their salts, preferably citric acid, citric heavy metal complexes for example silver citrate, triethylcitrate, benzoic acid, sodium benzoate, potassium benzoate, sorbic acid, potassium sorbate, sodium sorbate, ammonium sorbat, propionic acid, salicylic acid, salicylates, preferably sodium salicylate, ammonium salicylate, potassium salicylate, 2,4-hexandienic acid, methanoic acid, undecenylic acid, heterocyclic actives, preferably 3-acetyl-methyl-2,4-(3H)pyrandione, 5-amino-1,3-bis-(2-ethylhexyl)-5-methyl-hexahydropyrimidine, 4,4-dimethyl-1,3-oxazolidine, methylisothiazolinone, 1-hydroxy-4-methyl-6-(2,4,4-trimethylpentyl)-2(1H)-pyridone and its monoethanolamine salt, silver chloride, poly(hexamethylenediguanide)-hydrochloride, piroctose, preferably its zinc salt, pyrithiones and heavy metal salts thereof, preferably zinc pyrithione, zinc phenolsulfate, unsaturated antimicrobial actives, preferably farnesol, terbinafin or naftifine, heterocyclic aromatic actives, preferably bifonazole, cloconazole, isoconazole or diols, preferably pentanediol, caprylyl glycol, 1,2-hexanediol, ethylhexylglycerin, glyceryl caprylate and combinations of the antimicrobial actives.

The compositions according to the invention comprise the antimicrobial agents preferably in amounts of from 0.001 to 5 % by weight, particularly preferably in amounts of from 0.01 to 3 % by weight, particularly preferably in amounts of from 0.1 to 2 % by weight, based on the finished compositions.

Preferred astringents are oxides, preferably magnesium oxide, aluminum oxide, titanium dioxide, zirconium dioxide and zinc oxide, oxide hydrates, preferably aluminum oxide hydrate (boehmite) and hydroxides, preferably of calcium, magnesium, aluminum, titanium, zirconium or zinc.

The compositions according to the invention comprise the astringent active ingredients preferably in amounts of from 0 to 50 % by weight, particularly preferably in amounts of from 0.01 to 10 % by weight and especially preferably in amounts of from 0.1 to 10 % by weight, based on the finished compositions.

Advantageous compositions according to the invention comprise one or more antioxidants. Favorable, but nevertheless optional, antioxidants which can be used are all antioxidants which are customary or suitable for cosmetic and/or pharmaceutical application.

The antioxidants are advantageously chosen from the group consisting of amino acids (e.g. glycine, histidine, tyrosine, tryptophan) and derivatives thereof, imidazoles (e.g. urocanic acid) and derivatives thereof, peptides such as D,L-carnosine, D-carnosine, L-carnosine and derivatives thereof (e.g. anserine), carotenoids, carotenes (e.g. α-carotene, β-carotene, lycopene) and derivatives thereof, chlorogenic acid and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts), and sulfoximine compounds (e.g. buthionine sulfoximines, homocysteine sulfoximine, buthionine sulfones, penta-, hexa-, heptathionine sulfoximine) in very low tolerated doses (e.g. pmol/kg), and also (metal) chelating agents (e.g. α-hydroxyfatty acids, palmitic acid, phytic acid, lactoferrine), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. γ-linolenic acid, linoleic acid, oleic acid), folic acid and derivatives thereof, ubiquinone and ubiquinol and derivatives thereof, vitamin C and derivatives (e.g. ascorbyl palmitate, Mg ascorbyl phosphate, ascorbyl acetate), tocopherols and derivatives (e.g. vitamin E acetate), vitamin A and derivatives (e.g. vitamin A palmitate), and coniferyl benzoate of benzoin resin, rutinic acid and derivatives thereof, α-glycosylrutin, ferulic acid, furfurylideneglucitol, carnosine, butylhydroxytoluene, butylhydroxyanisole, nordihydroguaiacic acid, nordihydroguaiaretic acid, trihydroxybutyrophenone, uric acid and derivatives thereof, mannose and derivatives thereof, zinc and derivatives thereof (e.g. ZnO, ZnSO₄) selenium and derivatives thereof (e.g. selenomethionine), stilbenes and derivatives thereof (e.g. stilbene oxide, trans-stilbene oxide), superoxide dismutase and the derivatives (salts, esters, ethers, sugars, nucleotides, nucleosides, peptides and lipids) of these specified substances which are suitable according to the invention.

For the purposes of the present invention, water-soluble antioxidants can be used particularly advantageously.

The antioxidants can protect the skin and the hair against oxidative stress. Preferred antioxidants here are vitamin E and derivatives thereof, and vitamin A and derivatives thereof.

The amount of antioxidants (one or more compounds) in the compositions according to the invention is preferably 0.001 to 30 % by weight, particularly preferably 0.05 to 20 % by weight, in particular 1 to 10 % by weight, based on the finished compositions.

If vitamin E and/or derivatives thereof are the antioxidant or the antioxidants, it is advantageous to choose their particular concentrations from the range from 0.001 to 10 % by weight, based on the finished compositions.

In a particularly preferred embodiment of the invention, the cosmetic or pharmaceutical compositions comprise antioxidants chosen from superoxide dismutase, tocopherol (vitamin E) and ascorbic acid (vitamin C).

In a further preferred embodiment of the invention, the cosmetic or pharmaceutical compositions comprise one or more UV filters or UV absorbers. The one or more UV filters or UV absorbers may be inorganic or organic substances. These compositions may be sunscreen compositions for the protection of the skin and/or hair. However, these compositions may also be other compositions. In these other compositions the one or more UV filters or UV absorbers may protect the other ingredients of the compositions from the influence of UV radiation.

Pigments or micropigments which e.g. may be used as inorganic UV filters or UV absorbers are preferably microfine titanium dioxide, mica-titanium dioxide, iron oxides, mica-iron oxide, zinc oxide, silicon oxides, ultramarine blue, chromium oxides.

Suitable organic UV filters or UV absorbers are preferably 4-aminobenzoic acid; 3-(4'-trimethylammonium)-benzylideneboran-2-one methylsulfate; 3,3,5-trimethylcyclohexyl salicylate; 2-hydroxy-4-methoxybenzophenone; 2-phenylbenzimidazole-5-sulfonic acid and its potassium, sodium and triethanolamine salts; 3,3'-(1,4-phenylenedimethine)bis(7,7-dimethyl-2-oxobicyclo[2.2.1]heptane-1-methanesulfonic acid and its salts; 1-(4-tert-butylphenyl)-3-(4-methoxyphenyl)propane-1,3-dione, 3-(4'-sulfo)benzylidenebornan-2-one and its salts; 2-cyano-3,3-diphenylacrylic acid 2-ethylhexyl ester; polymer of N-[2(and 4)-(2-oxoborn-3-ylidenemethyl)benzyl]acrylamide; 4-methoxycinnamic acid 2-ethylhexyl ester; ethoxylated ethyl 4-aminobenzoate; 4-methoxycinnamic acid isoamyl ester; 2,4,6-tris[p-(2-ethylhexyloxycarbonyl)anilino]-1,3,5-triazine; 2-(2N-benzotriazol-2-yl)-4-methyl-6-(2-methyl-3-(1,3,3,3-tetramethyl-1-(trimethylsilyloxy)-disiloxanyl)propyl)phenol; 4,4'-[(6-[4-((1,1-dimethylethyl)aminocarbonyl)phenylamino]-1,3,5-triazin-2,4-yl)diimino]bis(benzoic acid 2-ethylhexyl ester); 3-(4'-methylbenzylidene)-D,L-camphor; 3-benzylidenecamphor; salicylic acid 2-ethylhexyl ester; 4-dimethylaminobenzoic acid 2-ethylhexyl ester; hydroxy-4-methoxybenzophenone-5-sulfonic acid (sulisobenzonum) and the sodium salt; and/or 4-isopropylbenzyl salicylate, N,N,N-trimethyl-4-(2-oxoborn-3-ylidenemethyl)anilinium methylsulfate, homosalate (INN) oxybenzone (INN), 2-phenylbenzimidazole-5-sulfonic acid and its Na, K and triethanolamine salts, alpha-(2-oxobom-3-ylidene)toluene-4-sulfonic acid and its salts, octylmethoxycinnamic acid, isopentyl-4-methoxycinnamic acid, isoamyl-p-methoxycinnamic acid, 2,4,6-trianilino-(p-carbo-2'-ethylhexyl-1'-oxy)-1,3,5-triazine (octyltriazone) phenol, 2-2(2H-benzotriazol-2-yl)-4-methyl-6-(2-methyl-3-(1,3,3,3-tetramethyl-1-(trimethylsilyl)oxy)disiloxanyl)propyl (drometriazole trisiloxane) benzoic acid, 4,4-((6-(((1,1-dimethylethyl)amino)carbonyl)phenyl)amino)-1,3,5-triazine-2,4-diyl)diimino)bis, bis(2-ethylhexyl)ester) benzoic acid, 4,4-((6-(((1,1-dimethylethyl)amino)carbonyl)phenyl)amino)-1,3,5-triazine-2,4-diyl)diimino)bis, bis(2-ethylhexyl)-ester) 3-(4'-methylbenzylidene)-d,l-camphor (4-methylbenzylidene camphor), 3-benzylidene camphor (3-benzylidene camphor), 2-ethylhexyl salicylate (octyl salicylate), ethyl-2-hexyl 4-dimethylaminobenzoate (octyldimethyl PABA), 2-hydroxy-4-methoxybenzophenone-5-sulfonic acid (benzophenone-5) and the Na salt, 2,2'-methylenebis-6-(2H-benzotriazol-2-yl)-4-(tetramethylbutyl)-1,1,3,3-phenol, sodium salt of 2-2'-bis(1,4-phenylene)1H-benzimidazole-4,6-disulfonic acid, (1,3,5)-triazine-2,4-bis((4-(2-ethylhexyloxy)-2-hydroxy)phenyl)-6-(4-methoxyphenyl), 2-ethylhexyl 2-cyano-3,3-diphenyl-2-propenoate, glyceryl octanoate di-p-methoxycinnamic acid, p-aminobenzoic acid and ester, 4-tert-butyl-4'-methoxydibenzoylmethane, 4-(2-[beta]-glucopyranoxy)propoxy-2-hydroxybenzophenone, octyl salicylate, methyl-2,5-diisopropylcinnamic acid, cinoxate, dihydroxydimethoxybenzophenone, disodium salt of 2,2'-dihydroxy-4,4'-dimethoxy-5,5'-disulfobenzophenone, dihydroxybenzophenone, 1-3,4-dimethoxyphenyl)-4,4-dimethyl-1,3-pentanedione, 2-ethylhexyl dimethoxybenzylidene dioxoimidazolidine propionate, tetrahydroxybenzophenone, terephthalidenedicamphorsulfonic acid, 2,4,6-tris[4-2-ethylhexyloxycarbonyl)anilino]-1,3,5-triazine, methylbis(trimethylsiloxy)silylisopentyltrimethoxycinnamic acid, amyl p-dimethylaminobenzoate, amyl p-dimethylaminobenzoate, 2-ethylhexyl p-dimethylaminobenzoate, isopropyl p-methoxycinnamic acid/diisopropylcinnamic acid ester, 2-ethylhexyl p-methoxycinnamic acid, 2-hydroxy-4-methoxybenzophenone, 2-hydroxy-4-methoxybenzophenone-5-sulfonic acid and the trihydrate, 2-hydroxy-4-methoxybenzophenone-5-sulfonate, Na salt, phenylbenzimidazolesulfonic acid.

The amount of the one or more inorganic or organic UV filters or UV absorbers in the compositions according to the invention is preferably 0.1 to 10 % by weight, particularly preferably 0.5 to 8 % by weight and especially preferably 1 to 5 % by weight, based on the finished compositions.

Suitable gelling agents are all surface-active substances which, dissolved in the liquid phase, form a network structure and thus consolidate the liquid phase. Suitable gelling agents are specified, for example, in WO 98/58625.

Preferred gelling agents are metal salts of fatty acids, preferably with 12 to 22 carbon atoms, for example sodium stearate, sodium palmitate, sodium laurate, sodium arachidate, sodium behenate, potassium stearate, potassium palmitate, sodium myristate, aluminum monostearate, hydroxyfatty acids, for example 12-hydroxystearic acid, 16-hydroxyhexadecanoyl acid; fatty acid amides; fatty acid alkanolamides; dibenzalsorbitol and alcoholic polyamides and polyacrylamides or mixtures thereof.

Preferably, the compositions according to the invention comprise 0.01 to 20 % by weight, particularly preferably 0.1 to 10 % by weight, especially preferably 1 to 8 % by weight and very particularly preferably 3 to 7 % by weight, of gelling agents, based on the finished compositions.

Further additives may be silicone compounds, preferably dimethylpolysiloxane, methylphenylpolysiloxanes, cyclic silicones, and amino-, fatty acid-, alcohol-, polyether-, epoxy-, fluorine- and/or alkyl-modified silicone compounds, for example alkylsilicones SilCare^{®} Silicone 41M10, SilCare^{®} Silicone 41M15, SilCare^{®} Silicone 41M20, SilCare^{®} Silicone 41M30 (Clariant), alkyltrimethicones SilCare^{®} 31M30, SilCare^{®} 31M40, SilCare^{®} 31M50, SilCare^{®} 31M60 (Clariant), phenyltrimethicones SilCare^{®} 15M30, SilCare^{®} 15M40, SilCare^{®} 15M50, SilCare^{®} 15M60 (Clariant), amodimethicones, for example SilCare^{®} Silicone SEA, polyalkylarylsiloxanes and polyethersiloxane copolymers.

The compositions according to the invention can comprise the abovementioned silicone compounds preferably in the amounts by weight of from 0.1 to 20 % by weight, particularly preferably 0.2 to 15 % by weight, especially preferably 0.5 to 10 % by weight, based on the finished compositions.

Suitable carrier materials are preferably vegetable oils, natural and hydrogenated oils, waxes, fats, water, alcohols, polyols, glycerol, glycerides, liquid paraffins, liquid fatty alcohols, sterol, polyethylene glycols, cellulose and cellulose derivatives.

The compositions according to the invention can advantageously be mixed with conventional ceramides, pseudoceramides, fatty acid N-alkylpolyhydroxyalkylamides, cholesterol, cholesterol fatty acid esters, fatty acids, triglycerides, cerebrosides, phospholipids and similar substances.

As pearlescence-imparting compounds, preference is given to fatty acid monoalkanolamides, fatty acid dialkanolamides, monoesters or diesters of alkylene glycol, in particular of ethylene glycol and/or propylene glycol or oligomers thereof with higher fatty acids, e.g. palmitic acid, stearic acid or behenic acid or mixtures thereof, monoesters or diesters of alkylene glycols with fatty acids, fatty acids and metal salts thereof, monoesters or polyesters of glycerol with carboxylic acids and ketosulfones of various types. In the compositions according to the invention, the pearlescence-imparting component is particularly preferably ethylene glycol distearate and polyethylene glycol distearate with 3 glycol units.

The moisturizing substances available are preferably isopropyl palmitate, glycerol and/or sorbitol, which are preferably used in the amounts by weight of from 0.1 to 50 %, based on the finished compositions.

Superfatting agents which may be used are preferably lanolin and lecithin, nonethoxylated and polyethoxylated or acylated lanolin and lecithin derivatives, polyol fatty acid esters, mono-, di- and triglycerides and/or fatty acid alkanolamides.

Dyes which can be used are the substances approved and suitable for cosmetic and pharmaceutical purposes. Examples of dyes which can be used are e.g. mentioned in ANNEX IV of the European Cosmetics Directive 76/768/EEC.

Fragrance and/or perfume oils which may be used are individual odorant compounds, e.g. the synthetic products of the ester, ether, aldehyde, ketone, alcohol and hydrocarbon type. Odorant compounds of the ester type are, for example, benzyl acetate, phenoxyethyl isobutyrate, p-tert-butylcyclohexyl acetate, linalyl acetate, dimethylbenzylcarbinyl acetate, phenylethyl acetate, linalyl benzoate, benzyl formate, ethyl methylphenylglycinate, allyl cyclohexylpropionate, styrallyl propionate and benzyl salicylate. The ethers include, for example, benzyl ethyl ether, and the aldehydes include, for example, the linear alkanals having 8 to 18 carbon atoms, citral, citronellal, citronellyloxyacetaldehyde, cycllamen aldehyde, hydroxycitronellal, lilial and bourgeonal, and the ketones include, for example, the ionones, alpha-isomethylionone and methyl cedryl ketone, and the alcohols include anethole, citronellol, eugenol, geraniol, linaloyl, phenylethyl alcohol and terpineol, and the hydrocarbons include primarily the terpenes and balsams. Preference is given to using mixtures of different odorants which together produce a pleasant scent note.

Perfume oils can also comprise natural odorant mixtures, as are accessible from vegetable or animal sources, e.g. pine, citrus, jasmine, lily, rose or ylang ylang oil.

Essential oils of lower volatility, which are mostly used as aroma components, are also suitable as perfume oils, e.g. sage oil, camomile oil, clove oil, melissa oil, mint oil, cinnamon leaf oil, linden blossom oil, juniper berry oil, vetiver oil, olibanum oil, galbanum oil and ladanum oil.

The acids or bases for adjusting the pH which are used are preferably mineral acids, in particular HCl, inorganic bases, in particular NaOH or KOH, and organic acids, in particular citric acid.

The compositions are preferably adjusted to a pH in the range from 2 to 12, preferably pH 3 to 9 and particularly preferably pH 4 to 8.

The cosmetic and pharmaceutical compositions according to the invention can be prepared using the polysiloxanes of the general formula (I).

The present invention therefore also provides the use of one or more of the polysiloxanes of the general formula (I) for preparing a cosmetic or pharmaceutical composition.

The polysiloxanes of the general formula (I) are preferably suited as emulsifiers. The present invention therefore is in also directed to the use of one or more polysiloxanes of the general formula (I) as emulsifier, preferably in the cosmetic or pharmaceutical compositions according to the invention, particularly preferably in the inventive emulsions.

The polysiloxanes of the general formula (I) are preferably suited for boosting the sun protection factor of a composition comprising one or more substances selected from the group consisting of inorganic and organic UV absorbers. The present invention therefore is also directed to the use of one or more polysiloxanes of the general formula (I) for boosting the sun protection factor of a composition comprising one or more substances selected from the group consisting of inorganic and organic UV absorbers, preferably in the cosmetic or pharmaceutical compositions according to the invention.

The polysiloxanes of the general formula (I) exhibit a softening effect for the skin and the hair. The invention thus also provides the use of a cosmetic or pharmaceutical composition according to the invention for softening the skin and/or the hair, preferably human skin and/or human hair.

The polysiloxanes of the general formula (I) exhibit an advantageous conditioning effect for the skin and/or the hair. The invention thus also provides the use of a cosmetic or pharmaceutical composition according to the invention for conditioning the skin and/or the hair, preferably human skin and/or human hair.

The polysiloxanes of the general formula (I) exhibit a volumizing and shine-imparting effect to hair, preferably human hair. The invention thus also provides the use of a cosmetic or pharmaceutical composition according to the invention, preferably in the form of a hairstyling composition, for imparting or improving volume and shine to the hair, preferably to human hair.

The polysiloxanes of the general formula (I) improve the color absorption behavior of hair colorants. At the same time, being color protection additives, they additionally improve the durability of hair tints or permanent hair colorants and improve the color stability of colored keratin fibers, preferably of colored human hair. The invention thus also provides the use of a cosmetic or pharmaceutical composition according to the invention for protecting and preserving the color in colored keratin fibers, preferably in colored human hair.

The polysiloxanes of the general formula (I) exhibit good skin sensory properties. The invention thus also provides the use of a cosmetic or pharmaceutical composition according to the invention for imparting good skin sensory properties, preferably to human skin.

The functionalized polysiloxane polymer of general formula (I) may be produced by first synthesizing an amino alkyl branched polysiloxane polymer of type (II). The conditions for the synthesis of these amino-substituted polysiloxanes of type (II) are known to the person skilled in the art. This reaction consists in an equilibrium reaction described in the silicone literature. The equilibrium leads to the insertion of amino alkyl silane units into the chain of a siloxane. The reaction is normally carried out in the presence of an acidic or alkaline catalyst.

Subsequently, the cyclic carbonate glycerine carbonate, is added to form the expected carbamate branched polysiloxane polymer of formula (I).

For example, the preparation of the above mentioned carbamate-functional polysiloxanes of general formula (I) can be carried out by reacting an amino-functional polysiloxane of general formula (II) 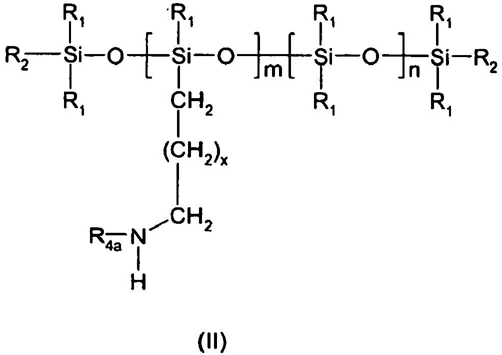 wherein R₁ and R₂ are defined as in formula (I); R₄ₐ is -CH₂-(CH₂)_{w}-NH₂; and m, n, w and x are defined as in formula (I),
with the following compound (D)

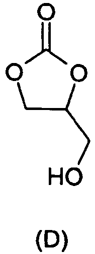

In the above general formula (II) independently
- R₁: represents a methyl residue;
- R₂: represents a methyl residue;
- m: has a value of from 1 to 100 and preferably an average value of from 1 to 10;
- n: has a value of from 1 to 3000 and preferably an average value of from 40 to 600; particularly preferably an average value of from 40 to 350;
- R₄ₐ: is -CH₂-(CH₂)_{w}-NH₂;
- w: is 1; and
- x: is 1.

The content of titrable nitrogen in the compounds of formula (II) is preferably An = 0.01 mmol/g to 2.0 mmol/g and particularly preferably 0.1 mmol/g to 1.0 mmol/g. An stands for amine number.

The branched amino-functional aminopolysiloxanes of general formula (II) preferably have an average viscosity of from 50 to 100,000 centipoises, especially from 100 to 15,000 centipoises at 25 °C.

The reaction may optionally be performed in the presence of a suitable solvent. Preferred are polar solvents, such as alcohols or ketones, example of which are methanol, ethanol, propanol, isopropanol, acetone or ethyl methyl ketone.

Preferably, the reaction is performed at temperatures within a range of from 40 to 120 °C, depending on the solvent employed.

More preferably, the reaction is performed at temperatures within a range of from 40 to 60 °C, without solvent.

The examples and applications below are intended to illustrate the invention in more detail without, however, limiting it thereto. All of the percentages given are percentages by weight (wt.-%) if not explicitly stated otherwise.

### Preparation examples of polyorganosiloxanes of general formula (I) and of comparative examples

Materials used in examples: Aminopropyl dimethoxysilane (APMDS), aminoethylaminopropyl diethoxysilane (DYNAS), Octamethylcyclotetrasiloxane (D4) and α,ω-OH-polydimethylsiloxane (WAPOSIL) are obtained from Wacker. Ethylene carbonate (EC), Glycerine carbonate (GC), Propylene carbonate (PC) and Butylene carbonate (BC) are obtained from Huntsman. Benzyltrimethylammonium hydroxide (40 % solution in methanol) are obtained from Aldrich. All materials are used as received without further purification.

For the aminosiloxane oils α,ω-terminated with OH groups, the synthesis is mainly done by reacting the specified amount of D4 or WAPOSIL and APMDS or DYNAS in the presence of benzyltrimethylammonium catalyst. The molecular weights of the oligomers are adjusted by changing the ratio of D4 to related amines. The reaction is run at 80 °C for 10 hours. After completion of the reaction, the temperature is increased to 170 °C and kept at that temperature for 1 hour to decompose the catalyst.

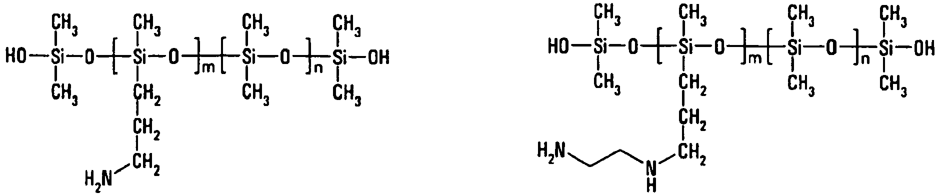

For the aminosiloxane oils α,ω -terminated with alkyl groups, the corresponding synthesis is mainly done by reacting the specified amount of WAPOSIL, hexamethyldisiloxane and APMDS or DYNAS in the presence of benzyl trimethylammonium catalyst. The molecular weights of the oligomers are adjusted by changing the ratio of hexamethyldisiloxane to related amines. As reported previously, the reaction is run at 80 °C for 10 hours. After completion of the reaction, the temperature is increased to 170 °C and kept at that temperature for 1 hour to decompose the catalyst.

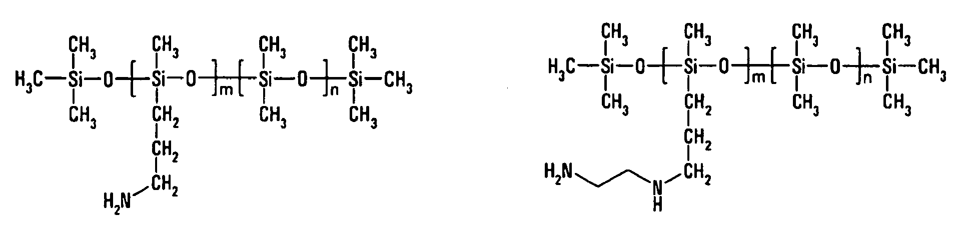

### Example 1

12.08 g (0.102 mol) of glycerine carbonate was added to 50 g of a polydimethylsiloxane α,ω-OH terminated containing aminopropyl groups having a content of titrable nitrogen of An = 2.04 mmol/g. This mixture was stirred at 40 °C for 240 minutes. The initially low-viscous mixture became white, clear and significantly more viscous after a short time. The content of titrable nitrogen at the end of the reaction was An = 0.08 mmol/g. Conversion rate : 96%.

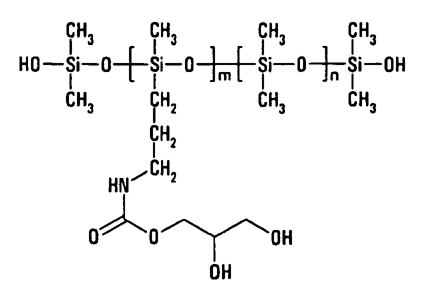

### Example 2

4.69 g (0.04 mol) of glycerine carbonate was added at room temperature to 50 g of a polydimethylsiloxane α,ω-methyl terminated containing aminoethylaminopropyl groups having a content of titrable nitrogen of An = 0.53 mmol/g. The mixture was stirred at 40 °C for 4 hours. The resulting white oil showed a final content of titrable nitrogen of An = 0.015 mmol/g. Conversion rate: 97 %

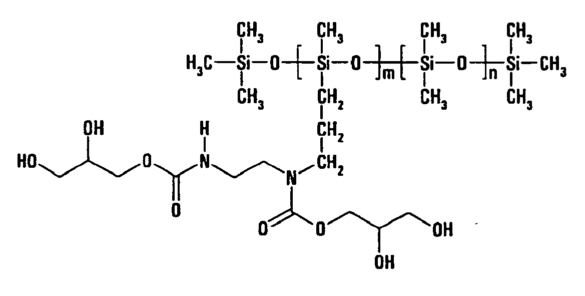

### Example 3

6.4 g (0.051 mol) of glycerine carbonate was added to 50 g of a polydimethylsiloxane α,ω-methyl terminated containing aminoethylaminopropyl groups and a content of titrable nitrogen of An = 0.98 mmol/g. The mixture was stirred at 60 °C for 90 minutes. The almost clear resulting oil was cooled to room temperature and its nitrogen content was measured at An = 0.06 mmol/g. Viscosity: 3620 Cps. Conversion rate: 94 %

**Table 1**

| Properties of aminoethylaminopropyl-polydimethylsiloxanes α,ω-methyl terminated reacted with glycerine carbonate. Anᵢ: initial amine content of the starting material. An_{f}: final amine content of the obtained polymer. The sum of the average values of the dimethylsiloxane units (n) and the methyl-amino siloxane units (m) is indicated in the table below as m + n. | | | | | | |
|---|---|---|---|---|---|---|
| Example | Anᵢ [mmol/g] | m + n | eq GC | Viscosity [Cps] | An_{f} [mmol/g)] | Appearance |
| 4 | 0.98 | 50 | 2 | 3020 | 0.06 | pale yellow |
| 5 | 0.54 | 300 | 2 | 1164000 | 0.02 | white |
| 6 | 0.55 | 170 | 2 | 12020 | 0.04 | white |
| 7 | 0.54 | 42 | 2 | 280 | 0.03 | white |

| | | | | | | |
|---|---|---|---|---|---|---|
| eq GC: equivalents glycerine carbonate with respect to amine groups of starting material according to formula (II) | | | | | | |

### Example 8

4.3 g (0.042 mol) of propylene carbonate was added to 10.06 g of a polydimethylsiloxane α,ω-methyl terminated containing aminopropyl groups having a content of titrable nitrogen of An = 2.0 mmol/g. This mixture was stirred at 40 °C for 240 minutes. The initially low-viscous mixture became white, clear and significantly more viscous after a short time. The content of titrable nitrogen at the end of the reaction was An = 0.02 mmol/g. Conversion rate: 99 %.

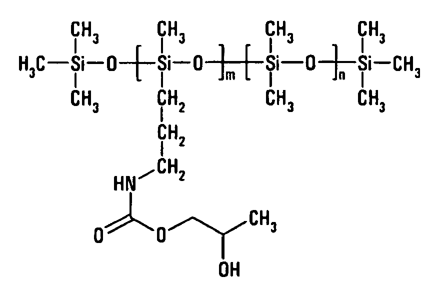

### Example 9 (comparative example)

4.8 g (0.042 mol) of butylene carbonate was added to 10.06 g of a polydimethylsiloxane α,ω-methyl terminated containing aminopropyl groups having a content of titrable nitrogen of An = 2.0 mmol/g. This mixture was stirred at 60 °C for 120 minutes. The initially low-viscous mixture became white, clear and significantly more viscous after a short time. The content of titrable nitrogen at the end of the reaction was An = 0.03 mmol/g. Conversion rate: 98 %.

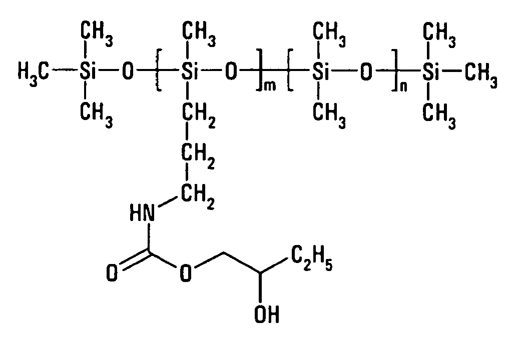

### Examples of cosmetic or pharmaceutical compositions

### Sunscreen for Children

| | | | |
|---|---|---|---|
| A | Cetiol OE | | 12.00 % |
| | Dicaprylyl Ether | | |
| | Titandioxid | | 10.00 % |
| | Titanium Dioxide | | |
| | Zinkoxid HP1 | | 8.00 % |
| | Zinc Oxide | | |
| | Miglyol 8810 | | 0.50 % |
| | Butylene Glycol Dicaprylate/Dicaprate | | |
| | Velsan^{®} CCT | (Clariant) | 6.00 % |
| | Caprylic/Capric Tryglyceride | | |
| | Velsan^{®} AB | (Clariant) | 6.00 % |
| | C₁₂₋₁₅ Alkyl Benzoate | | |
| | Dow Corning 245 | | 4.00 % |
| | Cyclomethicone | | |
| | Polysiloxane from example 2 | | 2.00 % |
| | Super Hartolan | | 0.50 % |
| | Lanolin Alcohol | | |
| | Trimethoxycaprylsilane | | 0.50 % |
| | Dehymuls PGPH | | 2.00 % |
| | Polyglyceryl-2 Dipolyhydroxystearate | | |
| | | | |
| B | Water | | ad 100.00 % |
| | 1,3-Butanediol | | 3.00 % |
| | Butylene Glycol | | |
| | Glycerin | | 3.00 % |
| | Magnesium sulfate-7-hydrate | | 0.80 % |
| | Magnesium Sulfate | | |
| | Glycin | | 0.70 % |
| | Panthenol | | 1.00 % |
| | Capsul | | 0.50 % |
| | Sodium Corn Starch Octenylsuccinate | | |
| | Elstab HP100 | | 0.50 % |
| | Hexamidine Diisethionate | | |
| | | | |
| C | Phenoxetol | (Clariant) | 0.60 % |
| | Phenoxyethanol | | |
| | Tocopheryl Acetat | | 0.50 % |

### Preparation:

I Phase A was heated up to 80 °C.
II Phase B was heated up to 80 °C.
III II was stirred into I and stirred until ambient temperature.
IV Components of C were added one after another to III at 35 °C.
V Finally the emulsion was homogenized.

### W/O Extra Moisture Cream for the Night

| | | | |
|---|---|---|---|
| A | Polysiloxane from example 2 | | 2.00 % |
| | Mineral Oil (30 mPa · s) | | 17.00 % |
| | Hydrogenated Castor Oil | | 0.40 % |
| | Microcrystalline Wax | | 0.60 % |
| | | | |
| B | Sodium Chloride | | 0.50 % |
| | Water | | ad 100 % |
| | | | |
| C | Urea | | 10.00 % |
| | Water | | 10.00 % |
| | | | |
| D | Nipaguard^{®} POM | (Clariant) | 0.80 % |
| | Phenoxyethanol and Piroctone Olamine and Methylparaben Parfum | | q.s. |

### Preparation:

I Phase A was heated up to 80 °C.
II Phase B was heated up to 80 °C and stirred into I until temperature was below 30 °C.
III Phase C was mixed and stirred into II.
IV Finally D was added to III and III was homogenized with a high shear mixer.

### W/O Silk Body Milk

| | | | |
|---|---|---|---|
| A | Polysiloxanes from examples 2, 3, 4, 6 or 7 | | 2.00 % |
| | Beeswax | | 0.50 % |
| | Hydrogenated Castor Oil | | 0.50 % |
| | Mineral Oil (30 mPa · s) | | 10.50 % |
| | Decyl Cocoate | | 8.00 % |
| | Tocopheryl Acetate | | 0.50 % |
| | Cyclomethicone | | 6.00 % |
| | | | |
| B | Glycerin | | 3.00 % |
| | Sodium Chloride | | 0.50 % |
| | Water | | ad 100 % |
| | | | |
| C | Alcohol | | 5.00 % |
| | Nipaguard^{®} PDU | (Clariant) | 0.60 % |
| | Propylene Glycol, Diazolidinyl Urea, Methylparaben, | | |
| | Propylparaben | | |
| | Parfum | | q.s. |

### Preparation:

I Phase A was heated up to 80 °C.
II Phase B was added to I slowly while stirring.
III II was homogenized for a short time.
IV After cooling with gentle stirring phase C was added below 40 °C to III.
V IV was homogenized again below 30 °C.

### Creamrinse

| | | | |
|---|---|---|---|
| A | Hostacerin^{®} DGI | (Clariant) | 1.50 % |
| | Polyglyceryl-2 Sesquiisostearate | | |
| | Cetearyl Alcohol | | 4.00 % |
| | | | |
| B | Genamin^{®} BTLF | (Clariant) | 1.50 % |
| | Behentrimonium Chloride | | |
| | Water | | ad 100 % |
| | | | |
| C | Fragrance | | 0.30 % |
| | Dyestuff solution | | q.s. |
| | Polysiloxane from example 2 | | 2.00 % |

### Preparation:

I Phase A was melted at about 70 °C.
II Phase B was heated to approximately 70 °C.
III II was added to I while stirring and stirred until cool.
IV At about 30 °C phase C was added to III.
V Finally the pH was adjusted to 4.0.

### Clear Conditioning Shampoo

| | | | |
|---|---|---|---|
| A | Genapol^{®} LRO liq | (Clariant) | 38.90 % |
| | Sodium Laureth Sulfate | | |
| | Water | | ad 100.00 % |
| | | | |
| B | Polysiloxane from example 2 | | 1.00 % |
| | Genagen^{®} CAB | (Clariant) | 15.00 % |
| | Cocamidopropyl Betaine | | |
| | Fragrance | | 0.30 % |
| | Preservative | | q.s. |
| | Genapol^{®} LA 030 | (Clariant) | 1.00 % |
| | Laureth-3 | | |
| | | | |
| C | Sodium Chloride | | 2.50 % |

### Preparation:

I The components of A were mixed.
II The components of B were added one after another to I.
III The pH was adjusted.
IV Finally the viscosity was adjusted with C.

### Perfume Spray

| | | | |
|---|---|---|---|
| A | Emulsogen^{®} LP | (Clariant) | 2.00 % |
| | Oleth- 5 | | |
| | Genapol^{®} C 100 | (Clariant) | 8.00 % |
| | Coceth-10 | | |
| | Mineral Oil, low viscosity | | 2.50 % |
| | Isopropylpalmitate | | 2.50 % |
| | Fragrance* | | 3.00 % |
| | | | |
| B | Polyglycol 400 | (Clariant) | 43.00 % |
| | PEG- 8 | | |
| | Polysiloxane from example 2 | | 2.00 % |
| | Water | | 37.00 % |

| | | | |
|---|---|---|---|
| * vanilla perfume recommended | | | |

### Preparation:

I The components of A were mixed.
II The components of B were added one after another to I and stirred.

### After Shave Balm Cream

| | | | |
|---|---|---|---|
| A | Polysiloxane from example 2 | | 4.00 % |
| | Isopropyl Isostearate | | 3.00 % |
| | Isopropyl Palmitate | | 2.00 % |
| | Abil^{®} 100 | | 1.00 % |
| | Dimethicone | | |
| | Menthol | | 0.10 % |
| | Campher | | 0.10 % |
| | | | |
| B | Aristoflex^{®} HMB | | 0.70 % |
| | Ammonium Acryloyldimethyltaurate/ | | |
| | Beheneth-25 Methacrylate Crosspolymer | | |
| | | | |
| C | Water | | ad 100 % |
| | Polyglycol 400 | (Clariant) | 3.00 % |
| | PEG-8 | | |
| | Extrapon Ginkgo Biloba | | 1.00 % |
| | Propylene Glycol, Water, Ginkgo Biloba Extract | | |
| | Extrapon Cucumber | | 1.00 % |
| | Water, Propylene Glycol, Cucumber (Cucumis Sativus) Extract, | | |
| | Phosphoric Acid | | |
| | Allantoin | (Clariant) | 0.30 % |
| | Allantoin | | |
| | | | |
| D | Nipaguard^{®} POM | (Clariant) | 0.50 % |
| | Phenoxyethanol, Piroctone Olamine, Methylparaben | | |
| | | | |
| E | Caustic soda solution (10 %) | | q.s. |

### Preparation:

I Phase A was melted at 60 °C, then phase B was added.
II Phase C was heated to 60 °C.
III II was stirred into I and stirred until cool.
IV Phase D was added to III at approximately 35 °C.
V Finally the pH was adjusted with E.

### O/W-Cream

| | | | |
|---|---|---|---|
| A | Hostacerin^{®} DGI | (Clariant) | 2.00 % |
| | Polyglyceryl-2 Sesquiisostearate | | |
| | Mineral Oil | | 8.00 % |
| | Eutanol^{®} G | | 4.00 % |
| | Octyldodecanol | | |
| | Isopropyl Palmitate | | 4.00 % |
| | Polysiloxane from example 2 | | 2.00 % |
| | | | |
| B | Aristoflex^{®} AVC | (Clariant) | 0.85 % |
| | Ammonium Acryloyldimethyltaurate/VP Copolmer | | |
| | (VP: Vinyl Pyrrolidone) | | |
| | | | |
| C | Hostapon^{®} KCG | (Clariant) | 0.70 % |
| | Sodium Cocoyl Glutamate | | |
| | Water | | ad 100.00 % |
| | Preservative | | q.s |
| | | | |
| D | Perfume | | 0.40 % |

### Preparation:

I Phases A and B were mixed.
II C was stirred into I.
III D was added to II.
IV Finally the emulsion was homogenized with a high shear mixer.

### Moisturizing Cream-Gel

| | | | |
|---|---|---|---|
| A | SilCare^{®} Silicone 31M50 | (Clariant) | 2.00 % |
| | Caprylyl Trimethicone | | |
| | Polysiloxane from example 2 | | 3.00 % |
| | Avocado Oil | | 4.00 % |
| | Tocopherol Acetate | | 0.50 % |
| | Phenonip^{®} | (Clariant) | q.s. |
| | Phenoxyethanol, Methylparaben, Ethylparaben, | | |
| | Butylparaben, Propylparaben, Isobutylparaben | | |
| | | | |
| B | Aristoflex^{®} AVL | (Clariant) | 4.10 % |
| | Caprylic/Capric Triglyceride (and) Ammonium Acryloyldimethyltaurate/VP Copolymer (VP: Vinyl Pyrrolidone) (and) Trilaureth-4 Phosphate (and) Polyglyceryl-2 Sesquiisostearate | | |
| | | | |
| C | Water | | ad 100 % |
| | | | |
| D | VitisVinifera (Grape) Seed Extract | | 0.05 % |
| | Dekluron^{®} | | 0.50 % |
| | Sodium Hyaluronate | | |
| | Glycerin | | 8.00 % |
| | | | |
| E | Fragrance | | 0.30 % |

### Preparation:

I The components of A were mixed.
II The components of D were dissolved in C.
III Phase B was stirred into I.
IV II was stirred into III.
V Phase E was stirred to IV and stirred well.
VI Finally the emulsion was homogenized.

### Sunscreen Cream Gel

| | | | |
|---|---|---|---|
| A | Eusolex^{®} 232 | | 8.00 % |
| | Phenylbenzimidazole Sulfonic Acid | | |
| | | | |
| B | Water | | ad 100 % |
| | | | |
| C | Aristoflex^{®} HMB | (Clariant) | 2.00 % |
| | Ammonium Acryloyldimethyltaurate/ | | |
| | Beheneth- 25 Methacrylate Crosspolymer | | |
| | | | |
| D | Velsan AB | (Clariant) | 5.00 % |
| | C₁₂₋₁₅ Alkylbenzoate | | |
| | SilCare^{®} Silicone 31M50 | (Clariant) | 2.00 % |
| | Phenyl Trimethicone | | |
| | Eusolex^{®} 9020 | | 3.00 % |
| | Butyl Methoxydibenzoylmethane | | |
| | Velsan^{®} D8P-3 | (Clariant) | 3.00 % |
| | Isopropyl PPG-2 Isodeceth-7 Carboxylate | | |
| | | | |
| E | Nipaguard^{®} MPA | (Clariant) | q.s. |
| | Benzyl Alcohol (and) Methylparaben (and) Propylparaben | | |
| | Genapol^{®} LA 070 | (Clariant) | 1.50 % |
| | Laureth-7 | | |
| | Polysiloxane from example 2 | | 3.00 % |
| | Fragrance | | q.s. |

### Preparation:

I Phases A and B were mixed and neutralized (pH to approximately 7.3).
II Phase C was added to I and stirred until a homogeneous gel was obtained.
III The components of D were mixed and after slightly heating the heated mixture was added to II.
IV Finally E was added to III.

### Hair Treatment

| | | | |
|---|---|---|---|
| A | Hostacerin^{®} DGI | (Clariant) | 1.50 % |
| | Polyglyceryl-2 Sesquiisostearate | | |
| | Cetearyl Alcohol | | 4.00 % |
| | Jojobaoil | | 1.00 % |
| | | | |
| B | Genamin^{®} BTLF | (Clariant) | 1.50 % |
| | Behentrimonium Chloride | | |
| | Genamin^{®} CTAC | (Clariant) | 2.00 % |
| | Cetrimonium Chloride | | |
| | | | |
| C | Water | | ad 100 % |
| | Preservative | | q.s. |
| | Hydrotriticum^{®} WQ | | 1.00 % |
| | Hydroxypropyltrimonium Hydrolyzed Wheat Protein | | |
| | Glycerin | | 2.00 % |
| | | | |
| D | Fragrance | | 0.30 % |
| | Dyestuff solution | | q.s. |
| | Polysiloxane from example 2 | | 1.50 % |

### Preparation:

I Phase A was melted at about 75 °C.
II Phase B was dissolved in phase C while stirring and heating to approximately 75 °C.
III II was added to I while stirring and stirred until cool.
IV At about 30 °C the components of D were added to III.
V Finally the pH was adjusted to 4.0

### Anti-Frizz

| | | | |
|---|---|---|---|
| A | Genapol^{®} T 200 | (Clariant) | 2.50 % |
| | Ceteareth-20 | | |
| | Water | | 11.00 % |
| | Glycerin | | 5.00 % |
| | | | |
| B | Polysiloxane from example 2 | | 3.00 % |
| | | | |
| C | Dow Corning^{®} 1501 Fluid | | 77.00 % |
| | Cyclopentasiloxane and Dimethiconol | | |
| | Isopropyl Palmitate | | 4.00 % |
| | Nipaguard^{®} POM | | 0.50 % |
| | Phenoxyethanol and Piroctone Olamine and Methylparaben | | |

### Preparation:

I The components of A were dissolved while stirring and heating slightly.
II Phase B was added to I.
III The components of C were mixed in a second beaker.
IV I was slowly added to III while homogenizing with a high shear mixer.

### Shimmering Bronze Gel

| | | | |
|---|---|---|---|
| A | Water | | ad 100 % |
| | Glycerin | | 5.00 % |
| | | | |
| B | Polysiloxane from example 2 | | 0.80 % |
| | Allantoin | (Clariant) | 0.20 % |
| | Allantoin | | |
| | | | |
| C | Aristoflex^{®} AVC | (Clariant) | 0.60 % |
| | Ammonium Acryloyldimethyltaurate/VP Copolymer | | |
| | (VP: Vinyl Pyrrolidone) | | |
| | Biron^{®} LF-2000 | | 3.00 % |
| | Bismuth Oxychloride | | |
| | Flamenco^{®} Ultra Silk | | 4.00 % |
| | Titanium oxide (and) Mica | | |
| | Gemtone^{®} Tan Opal | | 7.00 % |
| | Mica (and) Iron oxide (and) Titanium Dioxide | | |
| | Cloisonné^{®} Satin Bronze | | 5.00 % |
| | Iron oxide (and) Mica | | |
| | Gemtone^{®} Sunstone | | 2.00 % |
| | Mica (and) Iron oxide (and) Titanium oxide | | |
| | Desert Reflections ^{®} Sunlit Cactus | | 2.00 % |
| | Mica (and) Iron oxide (and) Titanium oxide (and) Tin oxide | | |
| | | | |
| D | Fragrance | | 0.15 % |
| | Nipaguard^{®} MPA | (Clariant) | q.s. |
| | Benzyl Alcohol (and) Methylparaben (and) Propylparaben | | |

### Preparation:

I Phase B was dissolved in A. No heating required.
II The components of C were mixed and added to I while stirring with low agitation.
III II was then stirred with higher agitation (approximately 200-250 r.p.m.) for roughly two hours to obtain a homogenous gel.
IV Phase D was added to III.

### Antiperspirant Roll-on

| | | | |
|---|---|---|---|
| A | Rewopol PEG 6000 DS | | 1.00 % |
| | PEG-150 Distearate | | |
| | | | |
| B | Water | | ad 100.00 % |
| | | | |
| C | Locron^{®} L | (Clariant) | 20.00 % |
| | Aluminum Chlorohydrate | | |
| | | | |
| D | Genapol^{®} T 250 | (Clariant) | 5.00 % |
| | Ceteareth-25 | | |
| | Butylene Glycol | | 3.00 % |
| | Cetiol OE | | 1.00 % |
| | Dicaprylyl Ether | | |
| | Glyceryl Isostearate | | 2.00 % |
| | | | |
| E | Polysiloxane from example 2 | | 0.50 % |

### Preparation:

I Phase A was mixed and dissolved in B while stirring and heating (80 °C).
II Phase C was added to I.
III Phase D was melted at approximately 50 °C and added to II while stirring. It was stirred until a clear solution was obtained.
IV Phase E was added at approximately 30 °C.

### O/W Soft Day Cream

| | | | |
|---|---|---|---|
| A | Hostaphat^{®} CC 100 | (Clariant) | 1.50 % |
| | Cetyl Phosphate | | |
| | Glyceryl Stearate | | 0.50 % |
| | Cetylstearyl Alcohol | | 0.50 % |
| | Mineral Oil, low viscosity | | 8.00 % |
| | Isopropyl Palmitate | | 7.00 % |
| | | | |
| B | Aristoflex^{®} AVC | (Clariant) | 1.20 % |
| | Ammonium Acryloyldimethyltaurate/VP Copolymer | | |
| | (VP: Vinyl Pyrrolidone) | | |
| | | | |
| C | Water | | ad 100 % |
| | Glycerin | | 5.00 % |
| | Sodium Hydroxide (10 % solution in water) 1.60 % | | |
| | Stay C 50 | | 0.05% |
| | Vitamin C | | |
| | Polysiloxane from example 3 | | 1.00 % |
| | Panthenol | | 0.50% |
| | Dekluron | | 0.05 % |
| | Sodium Hyaluronate | | |
| | | | |
| D | Tocopheryl Acetate | | 1.00 % |
| | Fragrance | | 0.20 % |
| | Nipaquard PDU^{®} | (Clariant) | 0.60% |
| | Propylene Glycol (and) Diazolidinyl Urea (and) | | |
| | Methylparaben (and) Propylparaben | | |

### Preparation

I Melt A at about 80 °C.
II Stir B into A.
III Dissolve the components of C at room temperature and add them to II.
IV At about 30 °C stir D into III.
V Finally homogenize the emulsion.

### Sunscreen Cream Gel

| | | | |
|---|---|---|---|
| A | Eusolex^{®} 232 | | 8.00 % |
| | Phenylbenzimidazole Sulfonic Acid | | |
| | | | |
| B | Water | | ad 100 % |
| | | | |
| C | Aristoflex^{®} HMB | (Clariant) | 2.00 % |
| | Ammonium Acryloyldimethyltaurate/ | | |
| | Beheneth- 25 Methacrylate Crosspolymer | | |
| | | | |
| D | Velsan AB | (Clariant) | 5.00 % |
| | C₁₂₋₁₅ Alkylbenzoate | | |
| | SilCare^{®} Silicone 15M50 | (Clariant) | 4.00 % |
| | Phenyl Trimethicone | | |
| | Eusolex^{®} 9020 | 3.00 % | |
| | Butyl Methoxydibenzoylmethane | | |
| | Velsan^{®} D8P-3 | (Clariant) | 3.50 % |
| | Isopropyl PPG-2 Isodeceth-7 Carboxylate | | |
| | | | |
| E | Nipaguard^{®} MPA | (Clariant) | q.s. |
| | Benzyl Alcohol (and) Methylparaben (and) Propylparaben | | |
| | Genapol^{®} LA 070 | (Clariant) | 1.50 % |
| | Laureth-7 | | |
| | Polysiloxane from example 8 | | 0.50 % |
| | Fragrance | | q.s. |

### Preparation:

I Mix A and B and neutralize it (adjust the pH to approximately 7.3).
II Add C and stir until a homogeneous gel has been obtained.
III Mix the components of D, dissolve them by slightly heating and add them to II.
IV Finally add E to III.

### Ultra Mild Antidandruff Shampoo

| | | | |
|---|---|---|---|
| A | Octopirox^{®} | (Clariant) | 0.50 % |
| | Piroctone Olamine | | |
| | | | |
| B | Water | | 10.00 % |
| | | | |
| C | Genapol^{®} LRO LIQUID | (Clariant) | 35.60% |
| | Sodium Laureth Sulfate | | |
| | | | |
| D | Hostapon^{®} KCG | (Clariant) | 4.60 % |
| | Sodium Cocoyl Glutamate | | |
| | Plantacare 818 UP | | 6.00 % |
| | Coco Glucoside | | |
| | Fragrance | | 0.30 % |
| | | | |
| E | Water | | ad 100.00 % |
| | | | |
| F | Celquat SC240C | | 0.30 % |
| | Polyquaternium 10 | | |
| | | | |
| G | Sodium Salicylate | | 1.00 % |
| | Dyestuff solution | | q.s. |
| | Genagen^{®} KB | (Clariant) | 8.00 % |
| | Coco Betaine | | |
| | Genapol^{®} DAT | (Clariant) | 1.00 % |
| | PEG-150 Polyglyceryl-2 Tristearate and | | |
| | PEG-6 Caprylic/Capric Glyceride | | |
| | Polysiloxane from example 9 | | 0.50 % |
| | | | |
| H | Sodium Chloride | | 0.40 % |

### Preparation:

I Mix A with B.
II Add C to I and keep stirring until a clear solution has been obtained.
III Add the components of D to I.
IV Dissolve the components of F in E while stirring and heating slightly, then add IV into I.
V Add the components of G into I.
VI Adjust the pH (pH 5.0-5.5).
VII Finally adjust the viscosity with H.

Examples relating to the use of compounds of formula (I) as emulsifier

### Example A

| Composition | wt.-% | wt.-% |
|---|---|---|
| Polysiloxane from example 2 | 1 | 3 |
| 50 % Dow Corning 245, 50 % Dow Corning 200 | 19 | 27 |
| Water | ad 100 % | ad 100 % |
| Viscosity [mPa · s] | 12200 | 4800 |
| Stability | stable | stable |

Dow Corning 245: Cyclomethicone; Dow Corning 200: Dimethicone

### Example B

| Composition | wt.-% |
|---|---|
| Polysiloxane from example 4 | 1 |
| 50 % Dow Corning 245, 50 % Dow Corning 200 | 19 |
| Water | ad 100 % |
| Viscosity [mPa · s] | 8600 |
| Stability | stable |

### Example C

| Composition | wt.-% | wt.-% |
|---|---|---|
| Polysiloxane from example 6 | 1 | 3 |
| 50 % Dow Corning 245, 50 % Dow Corning 200 | 19 | 27 |
| Water | ad 100 % | ad 100 % |
| Viscosity [mPa · s] | 9600 | 6200 |
| Stability | stable | stable |

### Example D

| Composition | wt.-% |
|---|---|
| Polysiloxane from example 7 | 1 |
| 50 % Dow Corning 245, 50 % Dow Corning 200 | 19 |
| Water | ad 100 % |
| Viscosity [mPa · s] | 5800 |
| Stability | stable |

### Example E

Test formulation:

| | | |
|---|---|---|
| A | Polysiloxanes from examples 2, 3, 4, 6 or 7 | 1.00 % |
| | Mineral oil (low viscosity) | 10.00 % |
| | Isopropyl Palmitate | 5.00 % |
| | Nipaguard^{®} PDU | 1.00 % |
| | Propylene Glycol and Methylparaben and | |
| | Propylparaben and Diazolidinyl Urea | |
| | | |
| B | Water | ad 100 % |
| | Glycerol | 3.00 % |

### Preparation:

I Components of phase A were mixed.
II Components of phase B were mixed.
III II was added to I and III was mixed until a homogeneous formulation was obtained.

Result: Stable, white and homogenous emulsion.

The polysiloxanes in examples A - E emulsify the water and the oil phase. Without the polysiloxanes stable emulsions have not been obtained.

## Claims

1. A cosmetic or pharmaceutical composition comprising one or more polysiloxanes of the general formula (I) 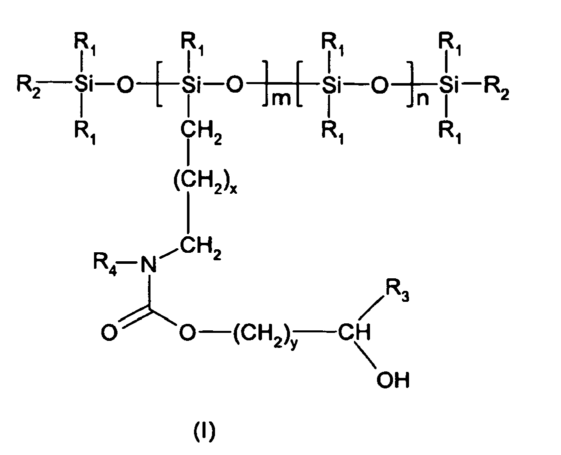 wherein
R₁ are methyl residues;
R₂ are methyl residues;
R₃ is a -CH₂-OH residue;
R₄ is -CH₂-(CH₂)_{w}-NH-C(=O)-O-(CH₂)_{z}-CH(OH)-R₅;
R₅ is a -CH₂-OH residue;
m has a value of from 1 to 100 and preferably an average value of from 1 to 10;
n has a value of from 1 to 3000 and preferably an average value of from 40 to 600, particularly preferably an average value of from 40 to 350;
w is 1;
x is 1;
y is 1; and
z is 1.

2. The cosmetic or pharmaceutical composition as claimed in claim 1, which comprises of from 0.01 to 10 % by weight, preferably of from 0.1 to 5 % by weight and particularly preferably of from 0.3 to 3 % by weight, based on the finished composition, of the polysiloxanes of the general formula (I).

3. The cosmetic or pharmaceutical composition as claimed in claim 1 or 2, which is a decorative cosmetic, preferably a make-up, eyeshadow, lipstick or a mascara, and comprises one or more substances selected from the group consisting of pigments, colorants, tints, dyes, pearlescent imparting substances and glitter imparting substances.

4. The cosmetic or pharmaceutical composition as claimed in one or more of claims 1 to 3, which is a sunscreen composition and comprises one or more substances selected from the group consisting of inorganic and organic UV absorbers.

5. The cosmetic or pharmaceutical composition as claimed in one or more of claims 1 to 4, which is an emulsion, preferably a water-in-oil- (W/O-) emulsion or an oil-in-water- (O/W-) emulsion, particularly preferably a water-in-oil-emulsion.

6. The use of one or more polysiloxanes of the general formula (I) 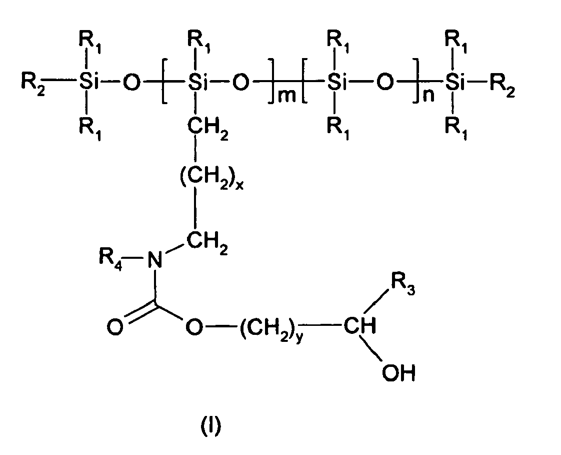 wherein
R₁ are methyl residues;
R₂ are methyl residues;
R₃ is a -CH₂-OH residue;
R₄ is -CH₂-(CH₂)_{w}-NH-C(=O)-O-(CH₂)_{z}-CH(OH)-R₅;
R₅ is a -CH₂-OH residue;
m has a value of from 1 to 100 and preferably an average value of from 1 to 10;
n has a value of from 1 to 3000 and preferably an average value of from 40 to 600, particularly preferably an average value of from 40 to 350;
w is 1;
x is 1;
y is 1; and
z is 1
as emulsifier in a composition as claimed in claim 5.

7. The use of one or more polysiloxanes of the general formula (I) 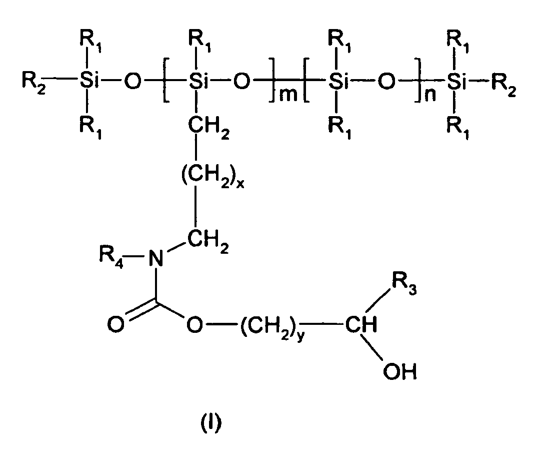 wherein
R₁ are methyl residues;
R₂ are methyl residues;
R₃ is a -CH₂-OH residue;
R₄ is -CH₂-(CH₂)_{w}-NH-C(=O)-O-(CH₂)_{z}-CH(OH)-R₅;
R₅ is a -CH₂-OH residue;
m has a value of from 1 to 100 and preferably an average value of from 1 to 10;
n has a value of from 1 to 3000 and preferably an average value of from 40 to 600, particularly preferably an average value of from 40 to 350;
w is 1;
x is 1;
y is 1; and
z is 1.
for boosting the sun protection factor of a composition as claimed in claim 4.

## Patentansprüche

1. Kosmetische oder pharmazeutische Zusammensetzung, umfassend ein oder mehrere Polysiloxane der allgemeinen Formel (I) 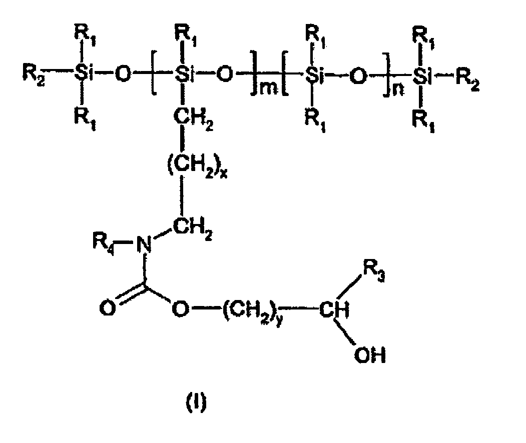 worin
R₁ für Methylreste stehen;
R₂ für Methylreste stehen;
R₃ für einen -CH₂-OH-Rest steht;
R₄ für -CH₂- (CH₂)_{w}-NH-C(=O)-O-(CH₂)_{z}-CH(OH)-R₅ steht;
R₅ für einen -CH₂-OH-Rest steht;
m einen Wert von 1 bis 100 und vorzugsweise einen durchschnittlichen Wert von 1 bis 10 aufweist;
n einen Wert von 1 bis 3000 und vorzugsweise einen durchschnittlichen Wert von 40 bis 600 und besonders bevorzugt einen durchschnittlichen Wert von 40 bis 350 aufweist;
w für 1 steht;
x für 1 steht;
y für 1 steht und
z für 1 steht.

2. Kosmetische oder pharmazeutische Zusammensetzung nach Anspruch 1, die 0,01 bis 10 Gew.-%, vorzugsweise 0,1 bis 5 Gew.-% und besonders bevorzugt 0,3 bis 3 Gew.-%, bezogen auf die fertige Zusammensetzung, der Polysiloxane der allgemeinen Formel (I) umfasst.

3. Kosmetische oder pharmazeutische Zusammensetzung nach Anspruch 1 oder 2, die ein dekoratives Kosmetikum, vorzugsweise ein Make-up, ein Lidschatten, ein Lippenstift oder ein Mascara ist und eine oder mehrere Substanzen aus der Gruppe bestehend aus Pigmenten, Farbmitteln, Tönungsmitteln, Farbstoffen, perlglanzgebenden Verbindungen und glittergebenden Verbindungen umfasst.

4. Kosmetische oder pharmazeutische Zusammensetzung nach einem oder mehreren der Ansprüche 1 bis 3, die eine Sonnenschutzzusammensetzung ist und eine oder mehrere Substanzen aus der Gruppe bestehend aus anorganischen und organischen UV-Absorbern umfasst.

5. Kosmetische oder pharmazeutische Zusammensetzung nach einem oder mehreren der Ansprüche 1 bis 4, die eine Emulsion, vorzugsweise eine Wasser-in-Öl-Emulsion (W/O-Emulsion) oder eine Öl-in-Wasser-Emulsion (O/W-Emulsion) und besonders bevorzugt eine Wasser-in-Öl-Emulsion ist.

6. Verwendung eines oder mehrerer Polysiloxane der allgemeinen Formel (I) 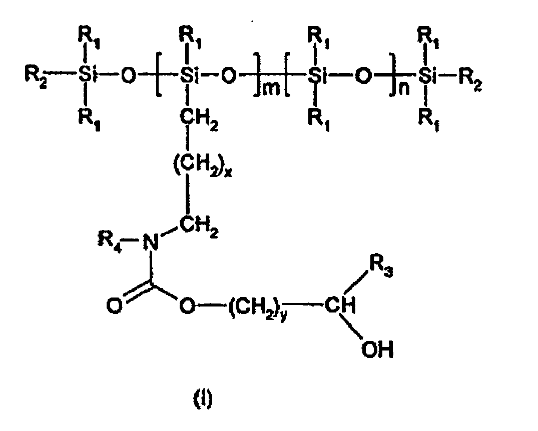 worin
R₁ für Methylreste stehen;
R₂ für Methylreste stehen;
R₃ für einen -CH₂-OH-Rest steht;
R₄ für -CH₂- (CH₂)_{w}-NH-C (=O) -O-(CH₂)_{z}-CH(OH)-R₅ steht;
R₅ für einen -CH₂-OH-Rest steht;
m einen Wert von 1 bis 100 und vorzugsweise einen durchschnittlichen Wert von 1 bis 10 aufweist;
n einen Wert von 1 bis 3000 und vorzugsweise einen durchschnittlichen Wert von 40 bis 600 und besonders bevorzugt einen durchschnittlichen Wert von 40 bis 350 aufweist;
w für 1 steht;
x für 1 steht;
y für 1 steht und
z für 1 steht;
als Emulgator in einer Zusammensetzung nach Anspruch 5.

7. Verwendung eines oder mehrerer Polysiloxane der allgemeinen Formel (I) 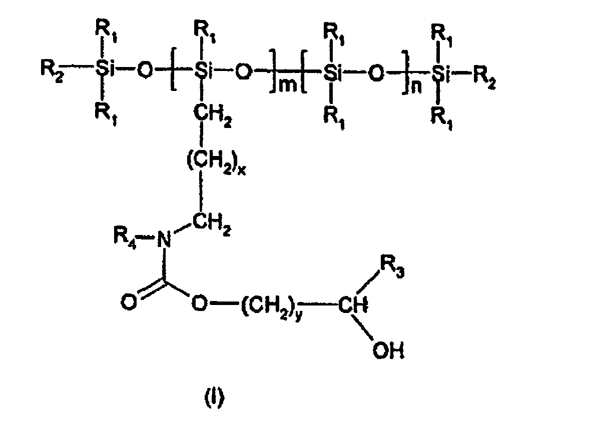 worin
R₁ für Methylreste stehen;
R₂ für Methylreste stehen;
R₃ für einen -CH₂-OH-Rest steht;
R₄ für -CH₂-(CH₂)_{w}-NH-C(=O)-O-(CH₂)_{z}-CH(OH)-R₅ steht;
R₅ für einen -CH₂-OH-Rest steht;
m einen Wert von 1 bis 100 und vorzugsweise einen durchschnittlichen Wert von 1 bis 10 aufweist;
n einen Wert von 1 bis 3000 und vorzugsweise einen durchschnittlichen Wert von 40 bis 600 und besonders bevorzugt einen durchschnittlichen Wert von 40 bis 350 aufweist;
w für 1 steht;
x für 1 steht;
y für 1 steht und
z für 1 steht;
zur Erhöhung des Sonnenschutzfaktors einer Zusammensetzung nach Anspruch 4.

## Revendications

1. Composition cosmétique ou pharmaceutique comprenant un ou plusieurs polysiloxanes de formule générale (I) 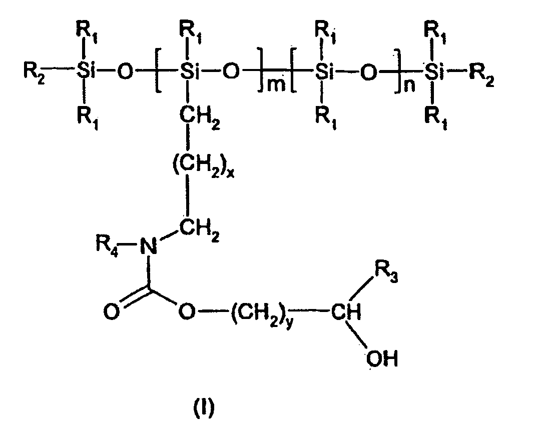 où
R₁ représentent des résidus méthyliques ;
R₂ représentent des résidus méthyliques ;
R₃ représente un résidu -CH₂-OH ;
R₄ représente -CH₂-(CH₂)_{w-}NH-C(=O)-O-(CH₂)_{z}-CH(OH)-R₅;
R₅ représente un résidu -CH₂-OH ;
m prend une valeur comprise entre 1 et 100 et préférentiellement une valeur moyenne comprise entre 1 et 10 ;
n prend une valeur comprise entre 1 et 3000 et préférentiellement une valeur moyenne comprise entre 40 et 600, particulièrement préférentiellement une valeur moyenne comprise entre 40 et 350 ;
w est égal à 1 ;
x est égal à 1 ;
y est égal à 1 ; et
z est égal à 1.

2. Composition cosmétique ou pharmaceutique conforme à la revendication 1, constituée d'entre 0,01 et 10 % en masse, préférentiellement d'entre 0,1 et 5 % en masse et particulièrement préférentiellement d'entre 0,3 et 3 % en masse, par rapport à la composition finie, de polysiloxanes de formule générale (I).

3. Composition cosmétique ou pharmaceutique conforme à la revendication 1 ou 2, qui est un produit cosmétique décoratif, préférentiellement un maquillage, un fard à paupières, un rouge à lèvres ou un mascara, et qui comprend une ou plusieurs substances choisies dans le groupe constitué par les pigments, les colorants, les teintes, les teintures, les substances nacrantes et les substances scintillantes.

4. Composition cosmétique ou pharmaceutique conforme à l'une quelconque des revendications 1 à 3, qui est une composition d'écran solaire et qui comprend une ou plusieurs substances choisies dans le groupe constitué par les agents absorbants UV inorganiques et organiques.

5. Composition cosmétique pharmaceutique conforme à l'une quelconque des revendications 1 à 4, qui est une émulsion, préférentiellement une émulsion eau dans l'huile (E/H) ou une émulsion huile dans l'eau (H/E), particulièrement préférentiellement une émulsion eau dans l'huile.

6. Emploi d'un ou de plusieurs polysiloxanes de formule générale (I) 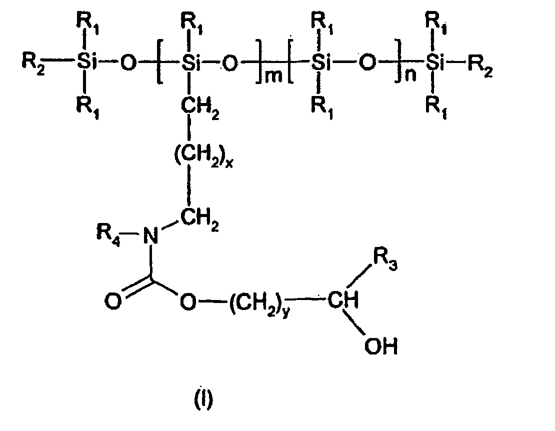 où
R₁ représentent des résidus méthyliques ;
R₂ représentent des résidus méthyliques ;
R₃ représente un résidu -CH₂-OH ;
R₄ représente -CH₂-(CH₂)_{w}-NH-C(=O)-O-(CH₂)_{z}-CH(OH)-R₅ ;
R₅ représente un résidu -CH₂-OH ;
m prend une valeur comprise entre 1 et 100 et préférentiellement une valeur moyenne comprise entre 1 et 10 ;
n prend une valeur comprise entre 1 et 3000 et préférentiellement une valeur moyenne comprise entre 40 et 600, particulièrement préférentiellement une valeur moyenne comprise entre 40 et 350 ;
w est égal à 1 ;
x est égal à 1 ;
y est égal à 1 ; et
z est égal à 1,
en tant qu'émulsifiant dans une composition conforme à la revendication 5.

7. Emploi d'un ou de plusieurs polysiloxanes de formule générale (I) 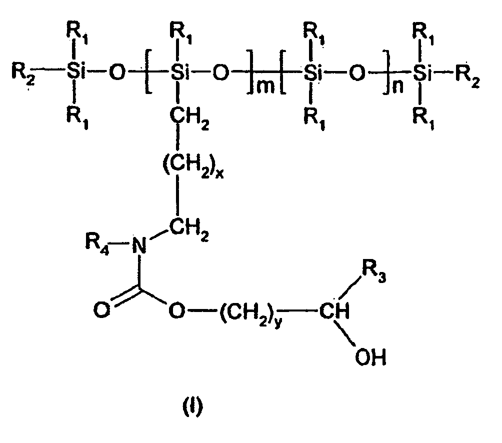 où
R₁ représentent des résidus méthyliques ;
R₂ représentent des résidus méthyliques ;
R₃ représente un résidu -CH₂-OH ;
R₄ représente -CH₂-(CH₂)_{w}-NH-C(=O)-O-(CH₂)_{z}-CH(OH)-R₅;
R₅ représente un résidu -CH₂-OH ;
m prend une valeur comprise entre 1 et 100 et préférentiellement une valeur moyenne comprise entre 1 et 10 ;
n prend une valeur comprise entre 1 et 3000 et préférentiellement une valeur moyenne comprise entre 40 et 600, particulièrement préférentiellement une valeur moyenne comprise entre 40 et 350 ;
w est égal à 1 ;
x est égal à 1 ;
y est égal à 1 ; et
z est égal à 1,
pour le renforcement de l'indice de protection solaire d'une composition conforme à la revendication 4.
